(19) Europäisches Patentamt
European Patent Office
Office européen des brevets

(11) EP 2 043 600 B1

(12) # FASCICULE DE BREVET EUROPEEN

(45) Date de publication et mention de la délivrance du brevet:
**26.07.2017 Bulletin 2017/30**

(21) Numéro de dépôt: **07730058.0**

(22) Date de dépôt: **11.06.2007**

(51) Int Cl.:
***A61K 9/00*** (2006.01) ***A61K 9/16*** (2006.01)
***A61K 9/10*** (2006.01) ***A61K 9/19*** (2006.01)
***A61K 38/27*** (2006.01) ***A61K 38/28*** (2006.01)
***A61K 38/21*** (2006.01)

(86) Numéro de dépôt international:
**PCT/EP2007/055720**

(87) Numéro de publication internationale:
**WO 2007/141344 (13.12.2007 Gazette 2007/50)**

(54) **FORMULATIONS PHARMACEUTIQUES POUR LA LIBERATION PROLONGEE DE PRINCIPE(S) ACTIF(S), AINSI QUE LEURS APPLICATIONS NOTAMMENT THERAPEUTIQUES**

PHARMAZEUTISCHE FORMULIERUNGEN FÜR DIE VERZÖGERTE FREISETZUNG VON WIRKSTOFF(EN) UND IHRE ANWENDUNGEN, INSBESONDERE THERAPEUTISCHE

PHARMACEUTICAL FORMULATIONS FOR THE SUSTAINED RELEASE OF ACTIVE INGREDIENT(S), AS WELL AS THEIR APPLICATIONS, ESPECIALLY THERAPEUTIC

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **09.06.2006 FR 0605152**

(43) Date de publication de la demande:
**08.04.2009 Bulletin 2009/15**

(73) Titulaire: **Flamel Ireland Limited**
**Dublin 15 (IE)**

(72) Inventeurs:
- **BONNET GONNET, Cécile**
**69004 Lyon (FR)**
- **CHOGNOT, David**
**33127 Martignas-sur-Jalle (FR)**
- **SOULA, Olivier**
**69330 Meyzieu (FR)**
- **CONSTANCIS, Alain**
**69003 Lyon (FR)**

(74) Mandataire: **Cabinet Plasseraud**
**235 Cours Lafayette**
**69006 Lyon (FR)**

(56) Documents cités:
**WO-A-00/30618** **WO-A-95/08320**
**WO-A-98/07410** **WO-A-2005/051416**
**WO-A2-2005/033181** **FR-A1- 2 801 226**
**US-A- 5 904 936**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 043 600 B1

## Description

La présente invention concerne de nouvelles formulations pharmaceutiques à base de suspensions colloïdales aqueuses pour la libération prolongée de principe(s) actif(s) (PA), en particulier protéinique(s) et peptidique(s), ainsi que les applications, notamment thérapeutiques, de ces formulations.

Ces formulations pharmaceutiques actives concernent aussi bien la thérapeutique humaine que vétérinaire.

Dans le domaine de la libération prolongée des PA pharmaceutiques notamment de protéines thérapeutiques, il existe un besoin, dans beaucoup de cas, de reproduire au mieux chez le patient une concentration plasmatique en protéine ou en peptide proche de la valeur observée chez le sujet sain.

Cet objectif se heurte à la faible durée de vie des protéines dans le plasma, ce qui conduit à injecter de façon répétée la protéine thérapeutique. La concentration plasmatique en protéine thérapeutique présente alors un profil "en dents de scie", caractérisé par des pics élevés de concentration et des minima de concentration très bas. Les pics de concentration, très supérieurs à la concentration basale chez le sujet sain, ont des effets nocifs très marqués du fait de la toxicité élevée des protéines thérapeutiques telles que l'interleukine IL2. Par ailleurs, les minima de concentration sont inférieurs à la concentration nécessaire pour avoir un effet thérapeutique, ce qui entraîne une mauvaise couverture thérapeutique du patient et des conséquences graves à long terme.

Aussi, pour reproduire chez le patient une concentration plasmatique en protéine thérapeutique proche de la valeur idéale pour le traitement du patient, il importe que la formulation pharmaceutique considérée permette de libérer la protéine thérapeutique sur une durée prolongée, de façon à limiter les variations de concentration plasmatique au cours du temps.

Par ailleurs, cette formulation active doit, de préférence, satisfaire au cahier des charges suivant, déjà connu de l'homme de l'art :

1 - libération prolongée d'une protéine thérapeutique active et non dénaturée, par exemple humaine ou synthétique, de sorte que la concentration plasmatique est maintenue au niveau thérapeutique ;
2 - viscosité à l'injection suffisamment basse pour être aisément injectable;
3 - forme biocompatible et biodégradable ;
4 - forme ne présentant ni toxicité, ni immunogénicité;
5 - forme ayant une excellente tolérance locale.

Pour tenter d'atteindre ces objectifs, l'une des meilleures approches proposées dans l'art antérieur fut de développer des formes à libération prolongée de protéine(s) thérapeutique(s) constituées par des suspensions, liquides et peu visqueuses de nanoparticules chargées en protéines thérapeutiques. Ces suspensions ont permis l'administration aisée de protéines thérapeutiques natives.

Ainsi, la société Flamel Technologies a proposé une voie, dans laquelle la protéine thérapeutique est associée à des nanoparticules d'un copolyaminoacide comprenant des groupements hydrophobes et des groupements hydrophiles.

Le brevet US-B-5,904,936 décrit des particules submicroniques (NPV) -de taille moyenne comprise entre 0,01 et 0,5 $\mu$m- et des particules micrométriques (MPV) -de taille moyenne comprise entre 0,5 et 20 $\mu$m- de copolymère amphiphile de polyaminoacides comprenant au moins deux types d'aminoacides, l'un étant neutre hydrophobe, l'autre étant ionisable. Des protéines telles que l'insuline s'adsorbent spontanément en solution aqueuse à ces particules. Le copolymère de polyaminoacide est, par exemple, un copolymère bloc de poly(L-leucine-b-L-glutamate de sodium). Ce brevet décrit l'agrégation de NPV en MPV par addition à une suspension colloïdale de poly-Leu/Glu, de sels monocationiques (sulfate d'ammonium) ou polycationiques ($Fe^{2+}$, $Fe^{3+}$, $Zn^{2+}$, $Ca^{2+}$, Ale, $Al^{3+}$ ou $Cu^{2+}$), d'acide (HCl), de polymères cationiques (polylysine).

La demande de brevet WO-A-2005/033181 divulgue des homopolyaminoacides linéaires, amphiphiles, anioniques, comprenant des unités aspartiques ou des unités glutamiques et dont les extrémités sont porteuses de groupements hydrophobes comportant de 8 à 30 atomes de carbone. En particulier, les homopolyaminoacides téléchéliques "modifiés hydrophobes" sont, par exemple, un poly[GluONa] à extrémités PheOC18/C18 ou un poly[GluONa] à extrémités PheOC18/alpha-tocophérol. Ces homopolyaminoacides téléchéliques "modifiés hydrophobes" forment spontanément dans l'eau une suspension colloïdale de nanoparticules, lesquelles sont aptes à s'associer aisément en suspension aqueuse à pH 7,4, avec au moins une protéine active (insuline).

La durée de libération *in vivo* de la (ou les) protéine(s) active(s) (par ex. insuline) "vectorisée" par les suspensions selon US-B-5,904,936 & WO-A-2005/033181 gagnerait à être augmentée.

L'augmentation de la durée de libération a été partiellement obtenue par les formes pharmaceutiques décrites dans la demande PCT WO-A-05/051416. Dans cette demande, une suspension colloïdale de nanoparticules (0,001-0,5 $\mu$m) de poly(L-glutamate de sodium) modifiée hydrophobe est injectée à une concentration telle qu'après injection sous-cutanée, il se forme *in situ* chez le patient un gel au contact de la sérumalbumine endogène. La protéine est alors

lentement libérée sur une période typiquement d'une semaine. Cependant, lorsque la concentration en protéine thérapeutique à administrer est relativement élevée, comme c'est le cas par exemple pour l'hormone de croissance humaine, la durée de libération est limitée à quelques jours seulement.

En tout état de cause, tout cet art antérieur sur les colloïdes de nanoparticules ou de microparticules de polyaminoacides modifiés hydrophobes ne révèle pas de formulation permettant :

(I) d'accroître la durée de libération de la protéine active après injection par voie parentérale, par exemple sous-cutanée; en particulier lorsque la concentration en protéine est élevée.
(II) et/ou de réduire le pic de concentration plasmatique de la protéine active après injection de la formulation la contenant.

Dans ces conditions, l'un des objectifs de la présente invention est donc de proposer une formulation pharmaceutique pour la libération prolongée de PA, remédiant aux carences de l'art antérieur, et en particulier permettant après injection par voie parentérale (par ex. sous-cutanée), d'obtenir une durée de libération *in vivo* prolongée pour des PA (par ex. protéines, peptides thérapeutiques et petites molécules) non dénaturés, par exemple des protéines humaines ou synthétiques.

Un autre objectif de l'invention est de proposer une formulation pharmaceutique permettant après injection par voie parentérale (par ex. sous-cutanée), d'obtenir une durée de libération *in vivo* prolongée pour des protéines et peptides thérapeutiques fortement concentrés, par exemple à plusieurs mg/ml.

Un autre objectif de l'invention est de proposer une formulation pharmaceutique à libération prolongée du PA *in vivo,* qui soit stable à la conservation tant sur le plan physico-chimique que biologique.

Un autre objectif de l'invention est de proposer une formulation pharmaceutique à libération prolongée du PA *in vivo,* qui présente au moins l'une des propriétés suivantes: biocompatibilité, biodégradabilité, atoxicité, bonne tolérance locale.

Un autre objectif de l'invention est de proposer une formulation pharmaceutique pour la libération prolongée lente de PA *in vivo,* comprenant des particules micrométriques de polymère (PO) auto-associées ou auto-associables à au moins un PA, le PO étant un polymère biodégradable, hydrosoluble, porteur de groupements hydrophobes (GH) et de groupements ionisables (GI), formant spontanément dans l'eau une suspension de nanoparticules colloïdales.

Un autre objectif de l'invention est de proposer une formulation pharmaceutique comprenant des particules micrométriques du polymère PO *vide supra*, capable de libérer, après injection par voie sous-cutanée, une protéine ou un peptide thérapeutique sur une durée plus longue que la durée de libération obtenue après administration de la même protéine mélangée en milieu aqueux du PA avec la suspension colloïdale du polymère PO.

Un autre objectif de l'invention est de proposer une formulation pharmaceutique de libération prolongée lente de PA *in vivo,* cette formulation comprenant des particules micrométriques de polymère PO auto-associées à au moins un PA, le polymère PO étant, par exemple, un polyaminoacide dont la chaîne principale est formée par des résidus aspartiques et/ou des résidus glutamiques, au moins une partie de ces résidus étant modifiée par greffage d'au moins un groupement hydrophobe GH, dans la chaîne et/ou en bout de chaîne, PO étant en outre biodégradable, hydrosoluble, et amphiphile.

Un autre objectif de l'invention est de proposer des produits dérivés et/ou des précurseurs de la formulation visée dans les objectifs sus énoncés.

Il est du mérite de la demanderesse d'avoir découvert que l'agrégation des nanoparticules de la formulation selon WO 05/051416 en particules micrométriques contenant des ions multivalents de polarité opposée à celle des groupements GI, dans des proportions bien définies, conduisait à une sélection d'une population spécifique de microparticules permettant une prolongation significative de la durée de libération du PA (par ex. protéine ou peptide) avec lequel ces microparticules sont associées.

Il est également du mérite de la demanderesse d'avoir découvert que certains ions multivalents conduisaient à une excellente tolérance des formulations selon l'invention.

D'où il s'ensuit que l'invention concerne une formulation pharmaceutique liquide pour la libération prolongée de PA, comprenant une suspension colloïdale, aqueuse, de viscosité dynamique à 20°C inférieure ou égale à 1000 mPa.s, à base de particules micrométriques de polymère (PO),

i. le polymère PO

- étant un copolymère amphiphile, biodégradable, hydrosoluble, où ledit copolymère est un polyaminoacide dont la chaîne principale est formée par des résidus aspartiques ou des résidus glutamiques, au moins une partie de ces résidus étant porteurs de groupements hydrophobes (GH) et de groupements hydrophiles ionisables (GI), au moins en partie ionisés, et
- formant spontanément une suspension colloïdale de nanoparticules dans l'eau, à pH = 7,0, en condition isotonique,

ii. lesdites particules étant aptes à s'associer spontanément de façon non covalente avec au moins un principe actif (PA), à pH = 7,0, en condition isotonique,

formulation caractérisée en ce que les particules micrométriques de PO ont une taille, mesurée dans un test T, comprise entre 1 et 70 µm, de préférence entre 2 et 40 µm et en ce qu'elle contient des ions multivalents de valence inférieure ou égale à 4, de préférence des ions divalents, des ions trivalents ou leurs combinaisons, de polarité opposée à celle des groupements ionisables GI du polymère,
lesdits ions multivalents ayant été ajoutés pour provoquer l'agrégation des nanoparticules de PO en particules micrométriques dans une quantité telle que le rapport r,

mesuré dans un test M, et répondant à la formule $r = n \times \frac{[IM]}{[GI]}$, où

- n est la valence desdits ions multivalents,
- [IM] est la concentration molaire en ions multivalents,
- [GI] est la concentration molaire en groupements ionisables GI,

est compris entre 0,3 et 10, de préférence entre 0,6 et 5,0 et, plus préférentiellement encore, entre 0,8 et 3,0.

Ces particules micrométriques sélectionnées selon l'invention sont issues de l'agrégation d'un grand nombre de nanoparticules de copolymère amphiphile. Cette agrégation est avantageusement provoquée par la présence d'ions multivalents de polarité opposée à celle des groupements ionisables GI (au moins en partie ionisés) portés par le copolymère. Ces ions multivalents de polarité opposée à celle des groupes GI du copolymère, en se complexant avec des chaînes de polymères appartenant à des nanoparticules distinctes, provoquent la floculation des nanoparticules de polymère sous forme de microparticules.
Le PA peut s'associer spontanément aux nanoparticules de copolymère PO avant leur agrégation en particules micrométriques et/ou à ces dernières pendant et/ou après l'agrégation.

En outre, les formulations selon l'invention sont non toxiques, bien tolérées localement.

Dans tout le présent exposé, à la différence des microparticules de taille micrométrique (micronique) de la formulation selon l'invention, le terme "particules submicroniques" ou "nanoparticules" désigne des particules de taille (mesurée dans un test T' décrit ci-après) supérieure ou égale à 1 nm et inférieure à 500 nm, de préférence comprise entre 5 et 250 nm.

Au sens de l'invention, le terme "protéine" désigne aussi bien une protéine qu'un peptide, qu'il s'agisse d'un oligopeptide ou d'un polypeptide. Cette protéine ou ce peptide peuvent être modifiés ou non, par exemple, par greffage d'un ou de plusieurs groupements polyoxyéthyléniques.

Le principe actif est désigné par l'abréviation PA dans tout le présent exposé ; PA vise au moins un principe actif.

Au sens de l'invention et dans tout le présent exposé, les termes "association" ou "associer" employés pour qualifier les relations entre un ou plusieurs PA et les polymères PO (les polyaminoacides) signifient que le ou les PA sont liés au(x) polymère(s) PO par une liaison non covalente, par exemple par interaction électrostatique et/ou hydrophobe et/ou liaison hydrogène et/ou gêne stérique.
Les vitesses angulaires de centrifugation sont exprimées en rpm (révolution par minute), unité équivalente au tour par minute (tr/min) : avec $1 \text{ rpm} = \frac{2\pi}{60} \text{ rad . s}^{-1}$

**<u>Test T de mesure de la taille des microparticules par diffraction laser:</u>**

<u>a- Test T1 dans le cas où les microparticules sont sous forme de dispersion aqueuse</u>

*1 Matériel et conditions opératoires*

| | |
|---|---|
| Granulomètre laser | Malvern Mastersizer 2000 |
| Module | Voie Liquide Hydro 2000SM |

(suite)

| | |
|---|---|
| Volume du fluide vecteur dispersant | 150 ml |
| Longueurs d'onde (bleu et rouge) | 466 et 632 nm |
| Vitesse d'agitation | 2400 tr/min |
| Domaine d'analyse | 0,02 μm à 2000 μm |
| Modèle optique (Théorie de Mie)<br>Valeurs des indices de réfraction utilisés :<br>Fluide dispersant (eau)<br>Polystyrène latex | <br><br>$m_{fluide}$ = 1,33 + i.0<br>$m_{polystyrène}$ latex = 1,59 + i.0 |
| Valeurs de l'obscuration pour déclencher l'analyse | Entre 5 % et 20 % |
| Temps d'acquisition | 10 s |

*2 Préparation de l'échantillon*

Pour préparer l'échantillon à analyser, il faut diluer 400 μL de l'échantillon à analyser dans un tube à essai de 5 mL avec 600 μL d'eau déminéralisée, puis passer la préparation au vortex pendant 10 s (10 ± 5).

*3 Analyse de l'échantillon*

Le fluide circulant stocké dans le système de dispersion voie liquide au repos est vidangé et remplacé par de l'eau déminéralisée fraîche. L'agitation du mobile Hydro2000SM est positionnée à 2400 tr/min.
Démarrer la mesure avec les conditions expérimentales précitées :

1- Alignement du faisceau laser,

2- Enregistrement des bruits de fond.

Après ces étapes, l'opérateur introduit l'échantillon à analyser de la façon suivante : ajouter au goutte à goutte (à la pipette pasteur) l'échantillon dilué jusqu'à ce que l'obscuration soit comprise entre 5 % et 20 % et lancer l'acquisition.
On obtient les données relatives au D50, qui est le diamètre en dessous duquel se trouvent 50 % des objets analysés.
Réaliser la moyenne du D50 de 3 mesures réalisées sur 3 préparations différentes.

<u>b-Test T2 dans le cas où les microparticules sont sous forme sèche</u>

*1 Matériel et conditions opératoires*

| | |
|---|---|
| Granulomètre laser | Malvern Mastersizer 2000 |
| Module | Voie Liquide Hydro 2000SM |
| Volume du fluide vecteur dispersant | 150 ml |
| Longueurs d'onde (bleu et rouge) | 466 et 632 nm |
| Vitesse d'agitation | 2000 tr/min |
| Domaine d'analyse | 0,02 μm à 2000 μm |
| Modèle optique (Théorie de Mie)<br>Valeurs des indices de réfraction utilisés :<br>Fluide dispersant (eau)<br>Polystyrène latex | <br><br>$m_{fluide}$ = 1,39 + i.0<br>$m_{polystyrène}$ latex = 1,59 + i.0 |
| Valeurs de l'obscuration pour déclencher l'analyse | Entre 5 % et 20 % |

(suite)

| | |
|---|---|
| Temps d'acquisition | 10 s |

*2 Préparation de l'échantillon*

- Préparer une solution de Span 80 à 0,1 % dans l'heptane (pour cela peser 0,01 g de poudre de Span 80 dans un flacon de 20 ml puis ajouter par pesée de l'heptane afin d'obtenir au final 10 g)
- Peser environ 6 mg de poudre dans un tube à essai de 5 mL,
- Ajouter 0,7 g d'heptane contenant 0,1 % de Span 80 dans le tube à essai,
- Placer le tube à essai pendant 2 min dans un bain à ultrasons afin de bien disperser la poudre.

*3 Analyse de l'échantillon*

Le fluide circulant stocké dans le système de dispersion voie liquide au repos est vidangé et remplacé par de l'heptane. L'agitation du mobile Hydro2000SM est positionnée à 2000 tr/min.
Démarrer la mesure avec les conditions expérimentales précitées :

1- Alignement du faisceau laser,

2- Enregistrement des bruits de fond.

Après ces étapes, l'opérateur introduit l'échantillon à analyser de la façon suivante : ajouter au goutte à goutte (à la pipette pasteur) l'échantillon dilué jusqu'à ce que l'obscuration soit comprise entre 5 % et 20 % et lancer l'acquisition.
On obtient les données relatives au D50, qui est le diamètre en dessous duquel se trouvent 50% des objets analysés.
Réaliser la moyenne du D50 de 3 mesures réalisées sur 3 préparations différentes.

**Test T' de mesure de la taille des nanoparticules par diffusion quasi-élastique de la lumière**

Le diamètre hydrodynamique moyen des particules de polymère PO selon l'invention est mesuré selon le mode opératoire Md défini ci-après :

Les solutions de PO sont préparées à des concentrations de 1 ou 2 mg/ml en milieu NaCl 0,15 M et laissées sous agitation pendant 24 h. Ces solutions sont ensuite filtrées sur 0,8-0,2 μm, avant d'être analysées en diffusion dynamique de la lumière grâce à un appareil de type Malvern Compact Goniometer System, fonctionnant avec un faisceau laser He-Ne de longueur d'onde 632,8 nm et polarisé verticalement. Le diamètre hydrodynamique des nanoparticules de polymère PO est calculé à partir de la fonction d'autocorrélation du champ électrique par la méthode des cumulants, comme décrit dans l'ouvrage « Surfactant Science Series » volume 22, Surfactant Solutions, Ed. R. Zana, chap. 3, M. Dekker, 1984.

**Test M de mesure de la teneur en ions multivalents par chromatographie ionique** :

On prend comme exemple celui de la quantification de l'ion multivalent $Mg^{2+}$. La méthode est sensiblement la même (seuls les témoins changent) pour la détermination des autres cations.

*1 Matériel et réactifs*

Chromatographe ionique

Système de chromatographie ionique Dionex ICS2500 équipé d'un détecteur de conductivité, d'un suppresseur cationique en fonctionnement interne et d'un four thermostaté.

Colonne (Dionex) : colonne Ion Pac CS16 5x250mm
Colonne de garde (Dionex) : colonne de garde Ion Pac CG16 5x50mm

Suppresseur cationique (Dionex) : CSRS - ULTRA II - 4mm
Flacon plastique pour chromatographie ionique (Dionex)
Eau déminéralisée (MilliQ)
Acide chlorhydrique HCl 0,1 N (Riedel de Haën)
Acide méthane sulfonique (MSA) (Aldrich)
Sulfate de l'ion multivalent : par exemple $MgSO_4$ (Aldrich)

*2 Préparation des solutions*

Solvant: HCl 0,01N

Dans une fiole de 1 L contenant environ 500 mL d'eau MilliQ, introduire 100 mL d'HCl 0,1 N. Compléter à 1 L avec de l'eau MilliQ et placer sous agitation magnétique.

Phase mobile : MSA 30 mM

Dans un flacon en plastique de 2 L pour chromatographie ionique, introduire 2 L d'eau MilliQ à l'aide d'une éprouvette graduée. Ajouter 3,89 mL de MSA avec une pipette automatique.
Placer sur la chaîne et commencer le bullage d'hélium pour mélanger et dégazer la phase.

*3 Préparations des témoins et des échantillons*

Préparation des témoins :

Préparer 3 témoins dans une gamme de concentration en $Mg^{2+}$ de 1 mg/L à 2,6 mg/L.

| | | $MgSO_4$ | HCl 0,01 N | |
|---|---|---|---|---|
| SM1 | Fiole de 100ml | 50 mg | Compléter à 100 ml | Agitation magnétique |
| SM2 | Fiole de 100ml | 80 mg | Compléter à 100 ml | Agitation magnétique |
| SM3 | Fiole de 100ml | 130 mg | Compléter à 100 ml | Agitation magnétique |

Préparer des dilutions des solutions mères

| | | SMx | HCl 0,01 N |
|---|---|---|---|
| T1 | Fiole de 100ml | 1 ml | Compléter à 100 ml |
| T2 | Fiole de 100ml | 1 ml | Compléter à 100 ml |
| T3 | Fiole de 100ml | 1 ml | Compléter à 100 ml |

Préparation des échantillons

Pour les formulations microparticulaires bien vortexer juste avant le prélèvement et mélanger à l'aide de la pipette automatique.
Effectuer au moins une dilution en adaptant la pesée et le volume de dilution afin d'obtenir une concentration en $Mg^{2+}$ attendue entre 1 mg/L et 2,6 mg/L. La dilution se fait dans HCl 0,01 N afin de précipiter le polymère. Procéder par dilutions successives si nécessaire.
Placer sous agitation magnétique pendant au moins 30 min.
Filtrer sur 0,45 $\mu$m avant de mettre en flacon.
Effectuer deux préparations par échantillon.

*4 Conditions de chromatographie ionique*

| | |
|---|---|
| Volume d'injection | 25 µl |
| Débit | 1 mL/min |
| Durée de l'analyse | 25 min |
| Détection | conductivité en µS/min |
| Eluant | MSA 30 mM |
| Température de colonne | 40 °C |
| Suppresseur | cationique |
| Courant | 87 mA |

*5 Exploitation des résultats*

Détermination de la droite d'étalonnage

La droite de régression est obtenue en considérant tous les témoins de la séquence. Le coefficient de corrélation doit être > 0,99.

L'équation de la droite est la suivante :

$$Y = a\,X + b$$

Avec

Y = aire du pic de magnésium
X = concentration de la solution témoin (mg/L)

Détermination de la teneur en ion magnésium dans les échantillons

$$M = \frac{(Y_{essai} - b) \times V_d}{a \times P_e}$$

$$M = \text{teneur en } Mg^{2+} \text{ (g/l)}$$

$Y_{essai}$ = aire du pic de magnésium pour l'essai
a = pente de la droite de calibration
b = ordonnée à l'origine de la droite d'étalonnage
$V_d$ = volume de dilution (ml)
$P_e$ = prise d'essai de l'échantillon (mg)

Le résultat final est la moyenne des 2 essais. Le coefficient de variation généralement accepté en chromatographie ionique est de 10 % mais celui-ci sera confirmé lors de la validation de la méthode.

Calcul du rapport r

Dans le protocole précédent, nous avons décrit la détermination de la teneur en magnésium. De la même façon on détermine par une méthode analogue la teneur T pour d'autres cations multivalents ($Ca^{2+}$, $Zn^{2+}$, $Al^{3+}$,etc.).

Une fois la valeur de M ainsi déterminée on en déduit la concentration molaire en ion multivalent IM :

$$[IM] = \frac{M}{M_{ion}}$$ où $M_{ion}$ est la masse molaire de l'ion multivalent en g/mol.

Connaissant la structure du polymère ionisable, en particulier son degré de polymérisation théorique DP, sa masse molaire théorique Mp et son taux de greffage hydrophobe (c'est-à-dire la fraction $\alpha_H$ des fonctions modifiées par des greffons hydrophobes), on peut calculer la concentration molaire en groupements ionisables pour une concentration C en polymère (exprimée en g/l) :

$$[GI] = (1-\alpha_H)\times\frac{C \times DP}{M_p}$$

On calcule alors $r = n \times \frac{[IM]}{[GI]}$ où n est la valence de l'ion multivalent.

De préférence, la formulation selon l'invention est caractérisée en ce que les particules micrométriques ont une densité apparente $d_{app}$ en polymère, mesurée dans un test D décrit ci-après, comprise entre 0,05 et 1,0, de préférence entre 0,07 et 0,7, de préférence entre 0,1 et 0,5.

**Test D de mesure de la densité apparente $d_{app}$**:

La suspension de microparticules de concentration C (mg/g) en polymère PO est homogénéisée par agitation magnétique. 2 ml de suspension de microparticules sont prélevés à l'aide d'une micropipette et placés dans un tube de centrifugation dont on a pris préalablement la tare. La suspension de microparticules placée dans le tube est alors pesée (masse m en g). Le tube est placé dans une centrifugeuse et laissé à centrifuger pendant 30 min à 8000 tr/min. Le surnageant est prélevé précisément avec des micropipettes adaptées. On en déduit le volume du sédiment $V_{sed}$ (en ml) : $V_{sed} = V_{tot} - V_{sur}$.
La densité apparente $d_{app}$ en polymère (en g/$cm^3$) est donnée par la formule :

$$d_{app} = \frac{\frac{m \times c}{1000}}{V_{sed}}$$

La suspension de particules micrométriques chargées en PA permet une prolongation particulièrement intéressante de la durée de libération du PA (par ex. protéine thérapeutique ou peptide) ainsi qu'une réduction du pic de concentration plasmatique.

La durée de libération du PA est notamment significativement augmentée par rapport à celle des formulations de l'art antérieur, en particulier celles décrites dans la demande WO 05/051416. La prolongation de la durée de libération de PA *in vivo* induite par les formulations selon l'invention est d'autant plus appréciable que les PA (par ex. protéines thérapeutiques) sont toujours pleinement bioactifs et non dénaturés.

Ainsi, les formulations selon l'invention ont pour caractéristique fonctionnelle avantageuse un accroissement de la durée $T_r$ de libération d'un PA donné, tel que mesuré dans un test L, par rapport à la durée $t_r$ de libération mesurée dans le même test L, d'une formulation identique ne contenant pas d'ions multivalents, cet accroissement étant de préférence tel que $T_r$ est supérieur ou égal à 1,1 × $t_r$, et, plus préférentiellement encore tel que $T_r$ est supérieur ou égal à 1,5 × $t_r$.

**Test L de mesure de la libération du PA à partir des particules micrométrigues selon l'invention.** :

Découper des carrés de 2 × 2 cm dans l'absorbant en polypropylène.
Préparer la solution de tampon phosphate, notée PBS, en dissolvant 1 tablette de PBS (Aldrich) dans 200 ml d'eau. On obtient 200 ml d'une solution contenant 0,01 M de tampon phosphate + 0,0027 M de chlorure de potassium et 0,137 M de chlorure de sodium.
Préparer la solution tamponnée d'albumine bovine à 30 mg/g, notée BSA, en dissolvant 6 g de Bovine Serum Albumin, fraction V (SAFC) dans 294 g de tampon phosphate PBS préparé précédemment.
Prévoir deux tubes Falcon de 50 ml contenant l'un du PBS et l'autre de la solution de BSA pour imbiber les carrés d'absorbant, et les conserver au réfrigérateur.

Mettre 4 ou 5 ml de ces solutions dans des tubes hermétiques de contenance 5 ml (prévoir 5 échantillons pour chacun des deux milieux). Les conserver au réfrigérateur.
Après 15 h d'attente, placer les tubes de 5 ml contenant les phases continues à 37 °C une heure avant.
Imbiber une heure les carrés, 5 dans le PBS, 5 dans la BSA (ou plus en cas de problème lors de l'injection) à 37°C.
Injecter 0,5 ml de formulation en 27G (seringue 1ml) au centre de chaque carré (parallèlement à sa surface) préalablement épongé légèrement sur du papier.
Repérer le côté par lequel on a injecté.

Faire ainsi pour les 10 carrés, que l'on n'immergera qu'ensuite dans les milieux continus afin de synchroniser toutes les cinétiques. Placer les carrés de façon à ce que l'endroit par lequel on a réalisé l'injection se trouve vers le haut du tube.
Placer les échantillons dans un portoir et mettre le tout à l'étuve à 37 °C, sous agitation. L'agitation influe fortement sur la cinétique de relargage, et doit donc être contrôlée : agitation sur 40 et 9 cm entre les deux barres bloquantes.
On prélève 100 µl de la phase continue à différents temps.

*Dosage de la protéine par chromatographie phase liquide HPLC*

On utilise deux phases éluantes :

- Phase A : 1260 ml d'eau / 680 ml d'acétonitrile / 60 ml de THF / 1,8 ml de TFA
- Phase B : 630 ml d'acétonitrile / 340 ml d'eau / 30 ml de THF / 0,8 ml de TFA

Le tableau suivant regroupe les conditions HPLC

| | | | |
|---|---|---|---|
| Instrument | Agilent 1100 équipé avec :<br>- Un passeur d'échantillons automatique réfrigéré<br>- Un détecteur UV et FLD<br>- Une intégration automatique | | |
| Colonne | Keystone BetaBasic-18<br>- 4,6 x 150 mm<br>- 3 µm<br>- taille de pores 150 Å | | |
| Gradient | Temps (min) | %A | %B |
| | 0 | 100 | 0 |
| | 15 | 50 | 50 |
| | 35 | 10 | 90 |
| | 36 | 100 | 0 |
| | 40 | 100 | 0 |
| Temps d'analyse | 40 min (+ 20 min de lavage entre les injections) | | |
| Débit | 1,5 ml/min | | |
| Température de colonne | 38 °C | | |
| Température des échantillons | Réfrigéré (4 °C) | | |
| Volume d'injection | 100 µl | | |
| Détection | Absorbance UV à 280 nm<br>Fluorescence à :<br>- 296 nm (excitation)<br>- 330 nm (émission) | | |

On trace alors la concentration en protéine dans le milieu continu en fonction du temps.

On lit sur cette courbe la quantité totale de protéine libérée lorsque la courbe atteint un plateau : cette valeur doit représenter au moins 40 % de la protéine introduite au départ pour que le test soit valable. On lit alors sur cette courbe le temps noté Tr comme étant celui nécessaire pour relarguer 50 % du PA introduit.

Suivant une caractéristique préférée, la formulation pharmaceutique liquide selon l'invention est caractérisée

en ce que les particules micrométriques de la suspension sont susceptibles d'être obtenues spontanément dans un liquide aqueux par addition, à une suspension de nanoparticules de polymère PO et éventuellement un PA, d'un sel contenant des ions multivalents, de préférence des ions divalents, de polarité opposée à celle des groupes GI du polymère PO.

Conformément à un mode préféré de réalisation, la formulation selon l'invention comprend un PA associé aux microparticules de PO.

Cette formulation a pour avantage d'être injectable par voie parentérale et d'être liquide dans les conditions d'injection.

Suivant l'invention, les qualificatifs "liquide", "basse" ou "très faible viscosité" correspondent, avantageusement, à une viscosité dynamique à 20 °C inférieure ou égale à 1000 mPa.s. De préférence, la viscosité dynamique de la formulation, mesurée à 20 °C, pour un gradient de cisaillement de 1000 $s^{-1}$, est préférablement inférieure ou égale à 500 mPa.s., de préférence comprise entre 2 et 200 mPa.s par exemple, comprise entre 1,0 et 100 mPa.s, voire entre 1,0 et 50 mPa.s.

**<u>Mesure de la viscosité dynamique</u>**

La mesure de référence pour la viscosité dynamique peut être réalisée, par exemple, à 20 °C à l'aide d'un rhéomètre AR1000 (TA Instruments) équipé d'une géométrie cône-plan (4 cm, 2°). La viscosité v est mesurée pour un gradient de cisaillement de 1000 $s^{-1}$.

Cette faible viscosité rend les formulations de l'invention aisément injectables par voie parentérale, en particulier par voie muqueuse, intramusculaire, sous-cutanée, intradermique, intrapéritonéale, intracérébrale ou dans une tumeur, entre autres.
La formulation selon l'invention peut aussi être administrée par voie orale, nasale, pulmonaire, vaginale, oculaire ou buccale.

Cet état liquide ou cette faible viscosité des formulations de l'invention existe aussi bien à des températures d'injection correspondant à des températures ambiantes, par exemple comprises entre 4 et 30 °C, qu'à la température physiologique.

Les polymères PO utilisés dans l'invention sont des polymères biodégradables, hydrosolubles et porteurs de groupements hydrophobes GH et de groupements GI ionisables . Les groupements hydrophobes peuvent être en nombre réduit vis-à-vis du reste de la chaîne et peuvent se situer latéralement à la chaîne ou intercalés dans la chaîne, et être répartis de façon aléatoire (copolymère statistique) ou répartis sous forme de séquences ou de greffons (copolymères blocs ou copolymères séquencés).

Selon un mode préféré de réalisation de l'invention, les groupements hydrophobes GH sont situés latéralement à la chaîne.

Selon l'invention, PO est choisi parmi les (co)polyaminoacides amphiphiles.
Au sens de l'invention et dans tout le présent exposé, le terme « polyaminoacide » couvre aussi bien les polyaminoacides naturels que les polyaminoacides synthétiques, ainsi que les oligoaminoacides comprenant de 10 à 20 résidus d'acide aminé au même titre que les polyaminoacides comprenant plus de 20 résidus.

De préférence, les polyaminoacides utilisés dans la présente invention sont des oligomères ou des homopolymères comprenant des résidus récurrents acide glutamique ou aspartique ou des copolymères comprenant un mélange de ces deux types de résidus d'acide aminé. Les résidus considérés dans ces polymères sont ceux d'acides aminés ayant la configuration D, L ou D/L et sont liés par leurs positions alpha ou gamma pour le résidu glutamate ou glutamique et alpha ou bêta pour le résidu aspartique ou aspartate.

Les résidus préférés de la chaîne polyaminoacide principale sont ceux ayant la configuration L et une liaison de type alpha.

Selon l'invention, PO est un polyaminoacide formé par des résidus aspartiques ou des résidus glutamiques, au moins une partie de ces résidus étant porteurs de greffons comportant au moins un groupement hydrophobe GH. Ces polyaminoacides sont notamment du type de ceux décrits dans la demande de brevet PCT WO-A-00/30618.

Selon une première possibilité, le (ou les) PO de la formulation sont définis par la formule générale (**I**) suivante :

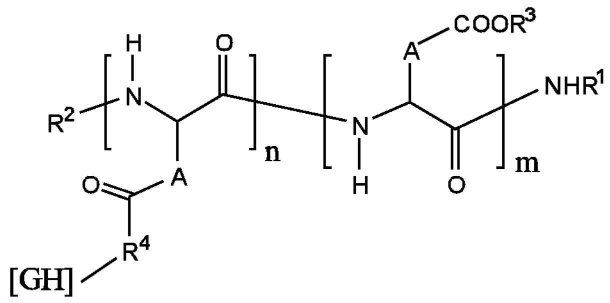

**(I)**

dans laquelle :

- $R^1$ représente un H, un alkyle linéaire en C2 à C10 ou ramifié en C3 à C10, un radical benzyle, un résidu acide aminé terminal, ou $-R^4$-[GH] ;
- $R^2$ représente un H, un groupe acyle linéaire en C2 à C10 ou ramifié en C3 à C10, un pyroglutamate ou $-R^4$-[GH] ;
- $R^3$ est un H ou une entité cationique, de préférence sélectionnée dans le groupe comprenant :

  - les cations métalliques avantageusement choisis dans le sous-groupe comprenant : le sodium, le potassium, le calcium, le magnésium,
  - les cations organiques avantageusement choisis dans le sous-groupe comprenant :

    - les cations à base d'amine,
    - les cations à base d'oligoamine,
    - les cations à base de polyamine (la polyéthylèneimine étant particulièrement préférée),
    - les cations à base d'acide(s) aminé(s) avantageusement choisis dans la classe comprenant les cations à base de lysine ou d'arginine,

  - ou les polyaminoacides cationiques avantageusement choisis dans le sous-groupe comprenant la polylysine ou l'oligolysine ;

- $R^4$ représente une liaison directe ou un espaceur à base de 1 à 4 résidus acide aminé ;
- A représente indépendamment un radical $-CH_2-$ (résidu aspartique) ou $-CH_2-CH_2-$ (résidu glutamique) ;
- n/(n+m) est défini comme le taux de greffage molaire et sa valeur est suffisamment basse pour que PO mis en solution dans l'eau à pH = 7 et à 25 °C, forme une suspension colloïdale de particules submicroniques de PO, de préférence n/(n + m) est compris entre 1 et 25 % molaire et mieux encore entre 1 et 15 % molaire ;
- (n + m) est défini comme le degré de polymérisation et varie de 10 à 1000, de préférence entre 50 et 300 ;
- GH représente un groupement hydrophobe.

Dans une forme préférée de réalisation de l'invention, la formulation est caractérisée en ce que le groupement hydrophobe GH est issu d'un précurseur alcoolique choisi dans le groupe comprenant: l'octanol, le dodécanol, le tétradécanol, l'hexadécanol, l'octadécanol, l'oléylalcool, le tocophérol ou le cholestérol, et en ce que $R^4$ représente une liaison directe.

Selon une deuxième possibilité, le (ou les) PO de la formulation répond(ent) à l'une des formules générales (**II**), (**III**) et (**IV**) suivantes :

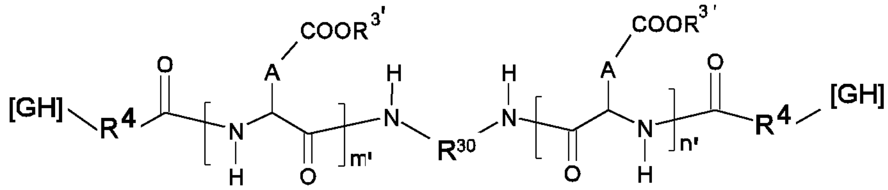

**(II)**

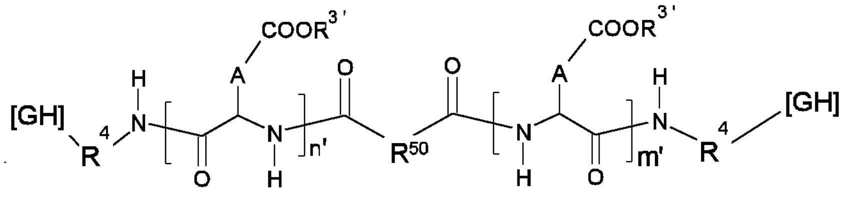

**(III)**

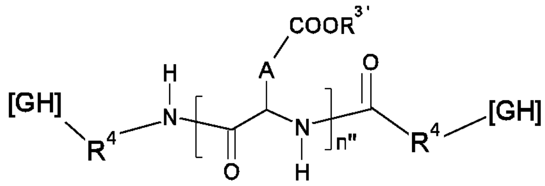

**(IV)**

dans lesquelles :

- GH représente un groupement hydrophobe ;
- $R^{30}$ est un groupement alkyle linéaire en C2 à C6 ;
- $R^{3'}$ est un H ou une entité cationique, de préférence sélectionnée dans le groupe comprenant :

  - les cations métalliques avantageusement choisis dans le sous-groupe comprenant : le sodium, le potassium, le calcium, le magnésium,
  - les cations organiques avantageusement choisis dans le sous-groupe comprenant :

    - les cations à base d'amine,
    - les cations à base d'oligoamine,
    - les cations à base de polyamine (la polyéthylèneimine étant particulièrement préférée),
    - les cations à base d'acide(s) aminé(s) avantageusement choisis dans la classe comprenant les cations à base de lysine ou d'arginine,

  - ou les polyaminoacides cationiques avantageusement choisis dans le sous-groupe comprenant la polylysine ou l'oligolysine,

- $R^{50}$ est un groupement alkyle, dialcoxy ou diamine en C2 à C6;
- $R^4$ représente une liaison directe ou un espaceur à base de 1 à 4 résidus acide aminé ;
- A représente indépendamment un radical $-CH_2-$ (résidu aspartique) ou $-CH_2-CH_2-$ (résidu glutamique) ;
- (n' + m') et n" sont définis comme le degré de polymérisation et varient de 10 à 1000, de préférence entre 50 et 300.

Avantageusement, les n groupements GH du PO représentent chacun indépendamment les uns des autres un radical monovalent de formule suivante :

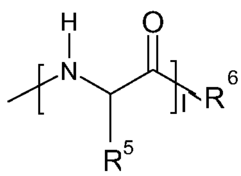

**(GH)**

dans laquelle :

- $R^5$ représente un radical méthyle (alanine), isopropyle (valine), isobutyle (leucine), secbutyle (isoleucine), benzyle (phénylalanine) ;
- $R^6$ représente un radical hydrophobe comportant de 6 à 30 atomes de carbone;
- 1 varie de 0 à 6.

Selon une caractéristique remarquable de l'invention, tout ou partie des groupements hydrophobes $R^6$ des PO sont choisis de façon indépendante, dans le groupe de radicaux comportant :

- un alcoxy linéaire ou ramifié comportant de 6 à 30 atomes de carbone et pouvant comporter au moins un hétéroatome (de préférence O, N ou S) ou au moins une insaturation,
- un alcoxy comportant 6 à 30 atomes de carbone et ayant un ou plusieurs carbocycles annelés et contenant éventuellement au moins une insaturation ou au moins un hétéroatome (de préférence O, N ou S),
- un alcoxyaryle ou un aryloxyalkyle de 7 à 30 atomes de carbone et pouvant comporter au moins une insaturation ou au moins un hétéroatome (de préférence O, N ou S).

En pratique et sans que cela ne soit limitatif, le radical hydrophobe $R^6$ du greffon du PO est issu d'un précurseur alcoolique choisi dans le groupe comprenant: l'octanol, le dodécanol, le tétradécanol, l'hexadécanol, l'octadécanol, l'oléylalcool, le tocophérol ou le cholestérol.

Avantageusement, la chaîne principale du polyaminoacide est :

► un homopolymère d'alpha-L-glutamate ou d'alpha-L-glutamique;
► un homopolymère d'alpha-L-aspartate ou d'alpha-L-aspartique
► ou un copolymère d'alpha-L-aspartate/alpha-L-glutamate ou d'alpha-L-aspartique/ alpha-L-glutamique.

De manière remarquable, la distribution des résidus aspartiques et/ou glutamiques de la chaîne polyaminoacide principale du PO est telle que le polymère ainsi constitué est soit aléatoire, soit de type bloc, soit de type multibloc.

Selon un autre mode de définition, le PO mis en oeuvre dans la formulation selon l'invention a une masse molaire qui se situe entre 2 000 et 100 000 g/mol, et de préférence entre 5 000 et 40 000 g/mol.

Selon une variante, le PO de la formulation selon l'invention est porteur d'au moins un greffon de type polyalkylène-glycol lié à un résidu glutamate ou aspartate.

Avantageusement, ce greffon est de type polyalkylène-glycol et de formule (V) suivante.

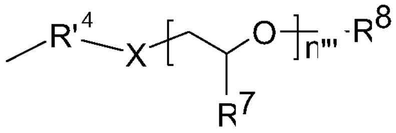

**(V)**

dans laquelle :

- $R^{14}$ représente une liaison directe ou un espaceur à base de 1 à 4 résidus acide aminé ;
- X est un hétéroatome choisi dans le groupe comportant l'oxygène, l'azote ou un soufre ;
- $R^7$ et $R^8$ représentent indépendamment un H, un alkyle linéaire en C1 à C4;
- n''' varie de 10 à 1000, de préférence de 50 à 300.

En pratique, le polyalkylène glycol est par exemple un polyéthylène glycol.

Il est souhaitable, conformément à l'invention, que le pourcentage molaire de greffage du polyalkylène glycol varie de 1 à 30 %.

Les polyaminoacides PO sont en outre extrêmement intéressants du fait qu'à un taux de greffage ajustable, ils se dispersent dans l'eau à pH = 7,4 (par exemple avec un tampon phosphate) pour donner des suspensions colloïdales.

De plus, des PA tels que des protéines, des peptides ou des petites molécules, peuvent s'associer spontanément à des nanoparticules comprenant ces polyaminoacides PO.

Il convient de comprendre que les PO contiennent des fonctions ionisables qui sont, selon le pH et la composition, soit neutres (par exemple COOH), soit ionisées (par exemple $COO^-$). Pour cette raison, la solubilité dans une phase aqueuse est directement fonction du taux de fonctions ionisées et donc du pH. En solution aqueuse, dans le cas de fonctions carboxyliques, le contre-ion peut être un cation métallique tel que le sodium, le calcium ou le magnésium, ou un cation organique tel que la triéthanolamine, le tris(hydroxyméthyl)-aminométhane ou une polyamine tel que la polyéthylèneimine.

Les PO de type polyaminoacide utilisés dans la formulation de l'invention sont, par exemple, obtenus par des méthodes connues de l'homme de l'art. Les polyaminoacides statistiques peuvent être obtenus par greffage du greffon hydrophobe, préalablement fonctionnalisé par l'espaceur, directement sur le polymère par une réaction classique de

couplage. Les PO polyaminoacides blocs ou multiblocs peuvent être obtenus par polymérisation séquentielle des anhydrides de N-carboxy-aminoacides (NCA) correspondants.

On prépare par exemple un polyaminoacide, homopolyglutamate, homopoly-aspartate ou un copolymère glutamate/aspartate, bloc, multibloc ou aléatoire selon des méthodes classiques.

Pour l'obtention de polyaminoacide de type alpha, la technique la plus courante est basée sur la polymérisation d'anhydrides de N-carboxy-aminoacides (NCA), décrite, par exemple, dans l'article *Biopolymers,* 1976, 15, 1869 et dans l'ouvrage de H.R. Kricheldorf "*alpha-Aminoacid-N-carboxy Anhydrides and relate Heterocycles*" Springer Verlag (1987). Les dérivés de NCA sont de préférence des dérivés NCA-O-Me, NCA-O-Et ou NCA-O-Bz (Me = méthyl, Et = éthyl et Bz = benzyl). Les polymères sont ensuite hydrolysés dans des conditions appropriées pour obtenir le polymère sous sa forme acide. Ces méthodes sont inspirées de la description donnée dans le brevet FR-A-2 801 226 de la demanderesse. Un certain nombre de polymères utilisables selon l'invention, par exemple, de type poly(alpha-L-aspartique), poly(alpha-L-glutamique), poly(alpha-D-glutamique) et poly(gamma-L-glutamique) de masses variables sont disponibles commercialement. Le polyaspartique de type alpha-bêta est obtenu par condensation de l'acide aspartique (pour obtenir un polysuccinimide) suivie d'une hydrolyse basique (cf. Tomida et al. Polymer 1997, 38, 4733-36).

Le couplage du greffon avec une fonction acide du polymère est réalisé aisément par réaction du polyaminoacide en présence d'un carbodiimide comme agent de couplage et optionnellement, un catalyseur tel que la 4-diméthylaminopyridine et dans un solvant approprié tel que le diméthylformamide (DMF), la N-méthyl pyrrolidone (NMP) ou le diméthylsulfoxide (DMSO). Le carbodiimide est par exemple le dicyclohexylcarbodiimide ou le diisopropylcarbodiimide. Le taux de greffage est contrôlé chimiquement par la stoechiométrie des constituants et réactifs ou le temps de réaction.

Les greffons hydrophobes fonctionnalisés par un espaceur sont obtenus par couplage peptidique classique ou par condensation directe par catalyse acide. Ces techniques sont bien connues de l'homme de l'art.

Pour la synthèse de copolymère bloc ou multibloc, on utilise des dérivés NCA préalablement synthétisés avec le greffon hydrophobe. Par exemple, le dérivé NCA-hydrophobe est copolymérisé avec le NCA-O-Bz puis on enlève par hydrolyse sélectivement les radicaux benzyles.

Les formulations selon l'invention, dans la forme de réalisation préférée où elles comprennent au moins un PA, résultent de l'association non covalente de nanoparticules à base de PO et d'au moins un PA, dans un milieu liquide aqueux.

Pour la préparation, le PO ou le PA peut être sous forme solide (de préférence poudre) ou sous forme liquide (de préférence suspension aqueuse colloïdale).

L'association PA/PO signifie au sens du présent exposé que le (ou les) PA est (sont) associé(s) au(x) polymère(s) PO [par ex. un ou plusieurs polyaminoacide(s)] par une ou plusieurs liaisons autre(s) qu'une (ou que des) liaison(s) chimique(s) covalente(s).

Les techniques d'association d'un ou de plusieurs PA aux PO selon l'invention, sont décrites notamment dans la demande de brevet WO-A-00/30618. Elles consistent à incorporer au moins un PA dans le milieu liquide contenant des nanoparticules de PO, de manière à obtenir une suspension colloïdale de nanoparticules chargées en ou associées avec un ou plusieurs PA.

Au sens de l'invention, le terme "ions multivalents" désigne des ions divalents, des ions trivalents, des ions tétravalents et des mélanges de ces ions.

Dans le cas où PO présente des groupements GI anioniques, les ions multivalents sont des cations multivalents, de préférence des cations divalents, plus préférentiellement encore choisis dans le groupe comprenant : $Mg^{2+}$, $Ca^{2+}$, $Zn^{2+}$, $Fe^{2+}$, $Cu^{2+}$ ou leurs mélanges, et/ou des cations trivalents, plus préférentiellement encore choisis dans le groupe comprenant : $Al^{3+}$, $Fe^{3+}$ ou leurs mélanges.

Il est possible qu'outre des ions multivalents, la formulation selon l'invention contienne des ions (par ex. cations) monovalents, éventuellement actifs dans l'agrégation des nanoparticules en microparticules.

Ces ions multivalents sont amenés dans la formulation de l'invention, de préférence sous forme de solution saline (organique ou minérale) aqueuse, par exemple une solution de sulfate, chlorure, acétate, gluconate ou glutamate (ou autre acide aminé anionique) de cations multivalents.

Pour améliorer sa stabilité, il est préférable que la formulation comprenne au moins un stabilisant sélectionné dans le groupe comprenant:

→ les nanoparticules d'au moins un polymère PO, PO étant un copolymère amphiphile, biodégradable, hydrosoluble et porteur de groupements hydrophobes (GH) et de groupements hydrophiles ionisables (GI), au moins en partie ionisés, et formant spontanément une suspension colloïdale de nanoparticules dans l'eau, à pH = 7,0, en condition isotonique,

→ les polyalkylène glycols, de préférence les polyéthylène glycols;

→ les copolyalkylène glycols, de préférence les copolymères éthylèneglycol-propylène-glycol (de type Poloxamer ou Pluronic ou Lutrol);

→ les polymères cellulosiques et leurs dérivés, de préférence les carboxyalkylcelluloses (par ex. les carboxyméthylcelluloses) ou les alkylcelluloses (par ex. les méthylcelluloses);

→ les esters de sorbitan et d'acide(s) gras, de préférence les esters de polyoxyalkylène (par ex. éthylène) glycol et d'au moins un acide (par ex. l'acide oléique), de type Tween ou Polysorbate;

→ les tensioactifs à base de phospholipides et de polyalkylène glycols, de préférence de polyéthylène glycols;

→ les saccharides hydrogénés ou non, tels que le tréhalose, le sorbitol, le mannitol, le saccharose;

→ des polyols tels que le propylène glycol ou le glycérol;

→ les gélatines de préférence hydrolysées;

→ les (co)polymères azotés, de préférence dans le groupe comprenant les polyacrylamides, les poly-N-vinylamides, les polyvinylpyrrolidones (PVP) et les poly-N-vinyl-lactames;

→ les alcools polyvinyliques (APV);

→ et leurs mélanges.

L'un des stabilisants préférés conformément à l'invention est formé par des nanoparticules d'au moins un polymère PO, identique (de préférence) ou différent de celui constituant les microparticules.
La quantité de stabilisant mis en oeuvre dans la formulation est de préférence comprise entre 0,01 et 10 % en poids, et plus préférentiellement encore entre 0,1 et 5 % en poids. S'agissant des stabilisants comprenant des nanoparticules, ils sont avantageusement utilisés dans la formulation à raison de 1,5 à 3,5 % en poids, par exemple de 2,0 à 3,0 (≈ 2,5) % poids.

Concernant le PA, il est de préférence choisi dans le groupe comprenant: les protéines, les glycoprotéines, les protéines liées à une ou plusieurs chaînes polyalkylèneglycol [de préférence polyéthylèneglycol (PEG) : "protéines-PEGylées"], les peptides, les polysaccharides, les liposaccharides, les oligonucléotides, les polynucléotides et leurs mélanges,
et, plus préférentiellement encore, dans le sous-groupe de érythropoïétine, ocytocine, vasopressine, hormone adrénocorticotrope, facteur de croissance épidermique, facteur de croissance plaquettaire (PDGF), facteurs de croissance hématopoïétiques et leurs mélanges, facteurs VIII et IX, hémoglobines, cytochromes, albumines, prolactine, hormone de libération de l'hormone lutéinisante (LHRH), antagonistes de la LHRH, agonistes de la LHRH, hormones de croissance (GH) humaine, porcine ou bovine, hormone de libération de l'hormone de croissance, insuline, somatostatine, glucagon, interleukines ou leurs mélanges (IL-2, IL-11, IL-12), interférons-α, β ou y, gastrine, tétragastrine, pentagastrine, urogastrone, sécrétine, calcitonine, enképhalines, endorphines, angiotensines, hormone de libération de la thyrotropine (TRH), facteurs de nécrose tumorale (TNF), facteurs neurotrophiques (NGF), facteur de croissance granulocytaire (G-CSF), facteur de croissance des granulocytes et macrophages (GM-CSF), facteur de croissance des macrophages (M-CSF), héparinase, protéine morphogénique osseuse (BMP), peptide auriculaire natriurétique (hANP), glucagon-like peptide 1 (GLP-1), facteur de croissance endothéliale vasculaire (VEGF), antigène recombinant de l'hépatite B (rHBs), rénine, cytokines, bradykinine, bacitracines, polymyxines, colistines, tyrocidine, gramicidines, cyclosporines et analogues synthétiques, modifications et fragments actifs pharmaceutiquement d'enzymes, de cytokines, d'anticorps, d'antigènes et de vaccins.

Selon une variante, le PA est une petite molécule organique hydrophobe, hydrophile ou amphiphile du type de celles appartenant à la famille des anthracyclines, des taxoïdes ou des camptothécines ou du type de celles appartenant à la famille des peptides telles que la leuprolide ou la cyclosporine et leurs mélanges
Au sens du présent exposé, une petite molécule est notamment une petite molécule non protéinique, par exemple exempte d'acides aminés.

Selon une autre variante, le PA est avantageusement choisi parmi au moins l'une des familles de substances actives suivantes : les agents de traitement de l'abus d'alcool, les agents de traitement de la maladie d'Alzheimer, les anesthésiques, les agents de traitement de l'acromégalie, les analgésiques, les antiasthmatiques, les agents de traitement des allergies, les agents anticancéreux, les anti-inflammatoires, les anticoagulants et antithrombotiques, les anticonvulsivants, les antiépileptiques, les antidiabétiques, les antiémétiques, les antiglaucomes, les antihistaminiques, les anti-infectieux, les antibiotiques, les antifongiques, les antiviraux, les antiparkinsoniens, les anti-cholinergiques, les antitussifs, les inhibiteurs de l'anhydrase carbonique, les agents cardiovasculaires, les hypolipémiants, les anti-arythmiques, les vasodilatateurs, les antiangineux, les anti-hypertenseurs, les vasoprotecteurs, les inhibiteurs de cholinestérase, les agents de traitement des désordres du système nerveux central, les stimulants du système nerveux central, les contraceptifs, les promoteurs de fécondité, les inducteurs et inhibiteurs du travail utérin, les agents de traitement de la mucoviscidose, les agonistes des récepteurs de la dopamine, les agents de traitement de l'endométriose, les agents de traitement des dysfonctionnements érectiles, les agents de traitement de la fertilité, les agents de traitements des troubles gastro-intestinaux, les immunomodulateurs et les immunosuppresseurs, les agents de traitement des troubles de la mémoire, les antimigraineux, les relaxants des muscles, les analogues de nucléosides, les agents de traitement de l'ostéoporose, les parasympathomimétiques, les prostaglandines, les agents psychothérapeutiques, les sédatifs, les hypnotiques et tranquillisants, les neuroleptiques, les anxiolytiques, les psychostimulants, les antidépresseurs, les agents de traitements dermatologiques, les stéroïdes et les hormones, les amphétamines, les anorexiques, les anti-douleurs

non analgésiques, les anti-épileptiques, les barbituriques, les benzodiazépines, les hypnotiques, les laxatifs, les psychotropes et toutes les associations de ces produits.

Sur le plan quantitatif, il est particulièrement intéressant que la fraction massique en PA non associé aux particules micrométriques [PA non associé] en % en poids soit telle que :

- [PA non associé] < 1
- de préférence [PA non associé] < 0,5.

Selon une variante, le PA peut être l'hormone de croissance humaine recombinante hGH, dont la dose est par exemple comprise entre 0,2 et 2 mg/kg, et de préférence entre 0,5 et 1 mg/kg.

Selon une autre variante, le PA peut être l'insuline, dont la dose est par exemple comprise entre 0,2 et 2 UI/kg, et de préférence entre 0,5 et 1 UI/kg.

Selon une autre variante, le PA peut être l'interferon $\alpha$-2b, dont la dose est par exemple comprise entre 20 et 100 $\mu$g/kg, et de préférence entre 40 et 80 $\mu$g/kg.

Avantageusement, la formulation selon l'invention est destinée à la préparation de médicaments, en particulier pour administration parentérale, muqueuse, sous-cutanée, intramusculaire, intradermique, intrapéritonéale, intracérébrale ou intra-tumorale, voire par voie orale, nasale, pulmonaire, vaginale ou oculaire.

Selon un autre de ses aspects, l'invention a pour objet un procédé de préparation de la formulation susvisée.

Ce procédé de préparation de la formulation est caractérisé en ce qu'il consiste essentiellement :

a. à mettre en oeuvre ou préparer une suspension colloïdale de nanoparticules d'au moins un PO;

b. éventuellement, à mélanger cette suspension colloïdale de nanoparticules de PO avec au moins un PA, de préférence en solution aqueuse;

c. éventuellement à filtrer la suspension ainsi obtenue;

d. à ajouter des ions multivalents (de préférence sous forme de sel(s)) de polarité opposée à celle des groupements GI du polymère PO, lesdits ions multivalents étant ajoutés en quantité telle que le rapport r, répondant à la formule $r = n \times \frac{[IM]}{[GI]}$, où :

- n est la valence desdits ions multivalents,
- [IM] est la concentration molaire en ions multivalents,
- [GI] est la concentration molaire en groupements ionisables GI,

est compris entre 0,3 et 10, de préférence entre 0,6 et 5,0 et, plus préférentiellement encore, entre 0,8 et 3,0;

e. au besoin à ajuster le pH ou l'osmolarité (par ex. par diafiltration).

La préparation des formulations liquides selon l'invention est avantageusement réalisée à température ambiante (25 °C par ex.).

Différents modes d'association du PA aux microparticules sont envisageables (*vide supra*). Il est avantageux que le PA soit associé aux nanoparticules destinées à former les microparticules par agrégation. Il est également possible d'associer le PA directement aux microparticules. Les deux méthodes d'association peuvent être combinées.

Pour l'association aux nanoparticules ou aux microparticules, le PA est avantageusement sous forme de suspension ou de solution aqueuse pour le mélange avec la suspension colloïdale de nanoparticules ou de microparticules de PO. Selon une variante, le PA sous forme solide pourrait être incorporé puis mélangé à la suspension de nanoparticules ou de microparticules.

La présente invention vise également des produits solides, dérivés des nanoparticules et des microparticules de PO contenu dans la formulation selon l'invention. En pratique, ces produits dérivés peuvent notamment être constitués par des poudres, des gels, des implants ou des films, entre autres.

Ainsi, l'invention vise des produits dérivés de la formulation selon l'invention, pris en tant que tels, quel que soit leur procédé d'obtention. Le produit dérivé en question est donc caractérisé en ce qu'il est sous forme non liquide et en ce qu'il comprend des particules micrométriques de polymère (PO),

i. le polymère PO

- étant un copolymère amphiphile, biodégradable, hydrosoluble, où ledit copolymère est un polyaminoacide dont

la chaîne principale est formée par des résidus aspartiques ou des résidus glutamiques, au moins une partie de ces résidus étant porteurs de groupements hydrophobes (GH) et de groupements hydrophiles ionisables (GI),
- et formant spontanément une suspension colloïdale de nanoparticules dans l'eau, à pH = 7,0, en condition isotonique,

ii. lesdites particules étant aptes à s'associer spontanément de façon non covalente avec au moins un PA, à pH = 7,0, en condition isotonique ;

ces particules micrométriques de PO ayant une taille, mesurée dans un test T, comprise entre 1 et 70 µm, de préférence entre 2 et 40 µm ;
et en ce qu'il contient des dérivés d'ions multivalents, de préférence d'ions divalents, de polarité opposée à celle des groupements GI du polymère, le rapport r,

répondant à la formule $r = n \times \frac{[IM]}{[GI]}$, où

- n est la valence desdits ions multivalents,
- [IM] est la concentration molaire en ions multivalents,
- [GI] est la concentration molaire en groupements ionisables GI,

étant compris entre 0,3 et 10, de préférence entre 0,6 et 5,0 et, plus préférentiellement encore, entre 0,8 et 3,0.
Ce produit dérivé peut, par exemple, être constitué par une poudre ou par un gel.
La présente invention vise également ces produits dérivés de la formulation selon l'invention en tant que produits intermédiaires issus de la préparation de la formulation selon l'invention. Cette dernière concerne donc aussi un procédé de préparation d'au moins l'un de ces produits dérivés. Ce procédé est caractérisé en ce qu'il consiste essentiellement à sécher la suspension de particules micrométriques, de manière à obtenir une forme solide, de préférence une poudre de particules micrométriques, susceptible d'être stockée ou administrée.
De tels produits dérivés donnent accès à une autre voie de préparation de la formulation selon l'invention. Le procédé selon cette voie est caractérisé en ce qu'il consiste essentiellement :

- à mettre en oeuvre au moins un produit dérivé obtenu par le procédé tel que défini *supra*,
- et à mélanger ce produit dérivé avec de l'eau ou une solution aqueuse S de reconstitution.

Dans ce dernier cas, la formulation pharmaceutique liquide est reconstituée extemporanément par mélange du produit dérivé solide (par ex. poudre) dans de l'eau ou dans S avant injection.
Par exemple, S peut comprendre une solution aqueuse. Il peut s'agir simplement d'eau pour préparations injectables. S peut contenir en plus éventuellement :

- au moins un tampon ou au moins un sel injectable (tampon phosphate, tampon citrate, chlorure de sodium) en concentration par exemple entre 0,001 M et 0,1 M de préférence entre 0,005 M et 0,02 M, ce tampon ou ce sel injectable permettant d'ajuster le pH de la solution;
- au moins un tensioactif injectable, de préférence de type polysorbate tels le Tween® 20 et le Tween® 80 ou de type poloxamer tels le lutrol® F38, le lutrol® F68 ou le lutrol® F127 dans des concentrations par exemple comprises entre 0,01 % et 2 % de préférence entre 0,05 et 0,5 %.

S peut contenir de plus des agents densifiants tels que des saccharides, à savoir le saccharose, le D-mannitol ou le tréhalose dans des concentrations comprises entre 0,1 % et 10 %, de préférence entre 0,5 et 5 %. La solution de reconstitution peut également contenir un polymère viscosifiant injectable choisi dans le groupe comprenant les polysaccharides, les polymères synthétiques (par ex. la carboxyméthylcellulose sodique), l'alcool polyvinylique, la polyvinylpyrrolidone, les polyalkylène glycols (par ex. polyéthylène glycols) et leurs mélanges.
Plus généralement, les excipients susceptibles d'être ajoutés à la formulation selon l'invention, sont par exemple des antimicrobiens, des tampons, des antioxydants, des agents permettant d'ajuster l'isotonicité qui sont connus de l'homme de l'art. On pourra par exemple se référer à l'ouvrage : Injectable Drug Development, P.K. Gupta et al., Interpharm Press, Denver, Colorado, 1999.
Conformément à l'invention, il est envisageable de prévoir une filtration stérilisante sur des filtres de porosité égale, par exemple, à 0,2 µm, de la suspension liquide de nanoparticules de laquelle sont issues les particules micrométriques de la formulation selon l'invention. L'agrégation en conditions stériles selon les modes de préparation décrits

ci-dessus permet ainsi d'injecter directement la formulation à un patient.

Parmi les qualités primordiales de la formulation selon l'invention figure sa capacité à libérer de façon prolongée le PA sur une durée supérieure à celle obtenue avec une suspension colloïdale de nanoparticules du même polymère, administrée au même pH, à la même concentration en protéine comme en polymère.

La présente invention a également pour objet un procédé de préparation de médicaments, en particulier pour administration parentérale, muqueuse, sous-cutanée, intramusculaire, intradermique, intrapéritonéale, intracérébrale ou intra-tumorale, voire par voie orale, nasale, pulmonaire, vaginale ou oculaire, caractérisé en ce qu'il consiste essentiellement à mettre en oeuvre au moins une formulation telle que définie ci-dessus *per se* ou une formulation obtenue par le procédé lui aussi défini ci-dessus, ou tout produit dérivé ou tout précurseur de ladite formulation.

Bien que la formulation selon l'invention soit de préférence pharmaceutique, cela n'exclut pas pour autant les formulations cosmétiques, diététiques ou phytosanitaires comprenant au moins un PO tel que défini ci-dessus et au moins un PA.

La formulation telle que décrite dans le présent exposé peut être administrée par voie parentérale, muqueuse, sous-cutanée, intramusculaire, intradermique, intrapéritonéale, intracérébrale ou dans une tumeur, voire par voie orale, nasale, pulmonaire, vaginale ou oculaire.

La formulation telle que décrite *supra* peut en particulier être administrée par injection parentérale, sous-cutanée, intramusculaire, intradermique, intrapéritonéale, intracérébrale
ou intra-tumorale, de préférence de manière à ce qu'elle forme un dépôt sur le site d'injection.

L'invention sera mieux comprise et ses avantages et variantes de mise en oeuvre ressortiront bien des exemples qui suivent et qui décrivent la synthèse des PO formés par des polyaminoacides greffés par un groupement hydrophobe, leur transformation en système de libération prolongée de PA, à savoir en formulation selon l'invention (suspension colloïdale aqueuse stable) et la démonstration de la capacité d'un tel système non seulement de s'associer à une protéine thérapeutique, mais surtout à gélifier/ réticuler pour libérer de manière très prolongée *in vivo* la protéine thérapeutique.

## DESCRIPTION DE LA FIGURE UNIQUE

La Figure unique est une courbe donnant le relargage de l'hormone de croissance humaine [hGH en % par rapport à la concentration totale injectée] en fonction du temps [t en min] dans le test L suivant qu'elle est incluse dans des nanoparticules de PO [■ nanoparticules 23 mg/g] ou des microparticules de PO [♦ microparticules 73 mg/g] préparées suivant l'exemple 8.

## EXEMPLES :

### Exemple 1 : Polymère amphiphile PO

Synthèse d'un polyglutamate greffé par l'alpha-tocophérol d'origine synthétique

On solubilise 15 g d'un acide alpha-L-polyglutamique (de masse équivalente à environ 16 900 Da par rapport à un standard en polyoxyéthylène et obtenu par polymérisation de NCAGluOMe suivie d'une hydrolyse comme décrits dans la demande de brevet FR-A-2 801 226) dans 288 ml de diméthylformamide (DMF) en chauffant à 80 °C jusqu'à solubilisation du polymère. On refroidit la solution à 15 °C et on ajoute successivement 2,5 g de D,L-alpha-tocophérol (> 98 % obtenu de Fluka®) préalablement solubilisé dans 8 ml de DMF, 280 mg de 4-diméthylaminopyridine préalablement solubilisée dans 1 ml de DMF et 1,6 g de diisopropylcarbodiimide préalablement solubilisé dans 6 ml de DMF. Après 3 h sous agitation, le milieu réactionnel est versé dans 1200 ml d'eau contenant 15 % de chlorure de sodium et d'acide chlorhydrique (pH = 2). Le polymère précipité est ensuite récupéré par filtration, lavé par de l'acide chlorhydrique 0,1 N, de l'eau et par de l'éther diisopropylique. Le polymère est ensuite séché à l'étuve sous vide à 40 °C. On obtient un rendement de l'ordre de 90 %. La masse molaire mesurée par chromatographie d'exclusion stérique est de 15 500 par rapport à un standard polyoxyéthylène. Le taux de tocophérol greffé, estimé par spectroscopie RMN du proton, est de 5,1 % molaire. Une suspension de nanoparticules du polymère dans l'eau est obtenue en le solubilisant dans l'eau puis en ajustant le pH (neutralisation des carboxylates) à 7 $\pm$ 1.

### Exemple 2 : Préparation de 100 g d'une suspension colloïdale de nanoparticules du polymère PO chargée en hGH

2.1 Préparation d'une solution colloïdale de polymère amphiphile PO:

On laisse une nuit le polymère en solution se thermostater à 30 °C.

On pèse 35,3 g de polymère PO de l'exemple 1.

On le dilue avec 26,65 g d'eau stérile pour injection (pour un polymère PO à 28,4 mg/g).

La solution de polymère est maintenue sous agitation magnétique.

L'osmolarité de la solution est ajustée à 300 ± 20 mOsm en introduisant 1,89 g d'une solution aqueuse de NaCl 5,13 M (30 % p/p).

Le pH est ajusté à 7,4 ± 0,2 par ajout de 0,38 g d'une solution de NaOH 1 N.

On obtient alors 64,22 g d'une solution de polymère à 15,61 mg/g.

2.2 Association de la protéine au polymère :

On décongèle à 25°C pendant 90 min la solution d'hormone de croissance humaine recombinante , notée hGH.

On ajoute ensuite 35,92 g de solution d'hGH (concentrée 3,9 mg/g) sur 64,15 g de la solution colloïdale de polymère préparée précédemment sous agitation.

La solution chargée en protéine est mise en maturation pendant 2 h à température ambiante.

Elle est ensuite filtrée sur 0,8-0,2 μm et laissée en maturation toute une nuit.

On obtient ainsi 100 g d'une formulation prête à être injectée contenant 1,4 mg/g d'hGH et 10 mg/g de polymère de type PO.

**Exemple 3: Préparation d'une suspension de particules micrométriques à l'aide de**

**$MgCl_2$**

La suspension est préparée à partir d'une solution préparée selon l'exemple 2, ajustée en pH et osmolarité et à 10,0 mg/g en PO.

a) Floculation de la solution par ajout d'une solution de $MgCl_2$ 1 M, sous agitation (position n°9) avec un pousse-seringue délivrant environ 20 ml/h. Le rapport caractérisant l'ajout de cations

$$r = 2 \times \frac{\lfloor Mg^{2+} \rfloor}{[Glu]}$$

est ici égal à 3,36. On obtient une solution à 8,51 mg/g de PO.

b) Centrifugation 25 min à 2500 rpm. Le surnageant limpide est à 646 mOsm.

c) Lavage du culot avec 1/8 du volume de la solution de départ d'eau stérile et centrifugation 10 min à 2500 rpm. Le surnageant limpide est à 350 mOsm.

d) Centrifugation 10 min à 2500 rpm.

Cette formulation est caractérisée comme suit

| | |
|---|---|
| pH | 6,64 |
| Osmolalité | 349 mOsm |
| [PO] | 50,77 mg/g |
| Diamètre des particules D50 (selon test T1) | 2μm |

**Exemple 4 : Obtention d'une suspension de microparticules fabriquées avec du $CaCl_2$ et avec diminution de pH**

La suspension est préparée à partir d'une solution préparée selon l'exemple 2, ayant pour caractéristiques finales 1,2 mg/g de hGH et 25,9 mg/g de PO

a) Ajustement à pH = 5,52 de la formulation initiale avec HCl 0,1 N sous agitation (600 rpm) avec un pousse-seringue délivrant environ 70 ml/h.

b) Floculation du polymère par ajout d'une solution de $CaCl_2$ 1 M sous agitation (600 rpm) avec un pousse-seringue délivrant environ 39 ml/h. Le rapport caractérisant l'ajout de cations

$$r = 2 \times \frac{\lfloor Ca^{2+} \rfloor}{[Glu]}$$

est ici égal à 1,68.

c) Centrifugation 8 min à 2500 rpm. Le surnageant limpide est à pH = 4,73 et à 653 mOsm.

d) Lavage du culot avec 1/8 du volume de la solution de départ d'eau milli-Q et centrifugation 4 min à 2500 rpm. Le surnageant limpide est à pH = 4,72 et à 378 mOsm.

e) Concentration du culot par centrifugation 4 min à 2500 rpm afin d'obtenir une suspension concentrée.

La suspension obtenue a les caractéristiques suivantes :

| | |
|---|---|
| pH | 4,88 |
| Osmolalité | 386 mOsm |
| [PO] | 122,88 mg/g |
| [hGH] | 4,9 mg/g |
| Diamètre des particules D50 | 23 µm |
| (selon test T1) | |

**Exemple 5: Obtention d'une suspension de microparticules fabriquées avec du $CaCl_2$**

La suspension est préparée à partir d'une solution préparée selon l'exemple 2, ayant pour caractéristiques finales 1,2 mg/g de hGH et 26,01 mg/g de PO.

a) Floculation de la formulation initiale par ajout d'une solution de $CaCl_2$ 1 M sous agitation (600 rpm) avec un pousse-seringue délivrant environ 40,3 ml/h jusqu'à atteindre un rapport r = 3,36. On obtient une formulation comprenant 0,93 mg/g de hGH pour 20,28 mg/g de PO.

b) Centrifugation 4 min à 2500 rpm. Le surnageant limpide est à pH = 6,34 et à 832 mOsm.

c) Lavage du culot avec 1/3 du volume de la solution de départ d'eau milli-Q et centrifugation 8 min à 2500 rpm. Le surnageant limpide est à pH = 6,56 et à 334 mOsm.

d) Concentration du culot par centrifugation 8 min à 2500 rpm afin d'obtenir une suspension concentrée.

Cette formulation est caractérisée comme suit:

| | |
|---|---|
| pH | 6,25 |
| Osmolalité | 343 mOsm |
| [PO] | 101,87 mg/g |
| [HGH] | 4,7 mg/g |
| Diamètre des particules D50 (selon test T1) | 13 µm |

**Exemple 6 : Obtention d'une suspension de microparticules fabriquées avec du $ZnCl_2$**

La suspension est préparée à partir d'une solution préparée selon l'exemple 2, ayant pour caractéristiques finales 1,4 mg/g de hGH et 10,00 mg/g de PO.

a) Floculation de la solution par ajout d'une solution de $ZnCl_2$ 1 M, sous agitation (position n°9) avec un pousse-seringue délivrant environ 20 ml/h jusqu'à obtention d'un rapport r = 1,54. On obtient une solution à 9,64 mg/g de PO.

b) Centrifugation 4 min à 2500 rpm. Le surnageant limpide est à 389 mOsm.

c) Lavage du culot avec 1/13 du volume de la solution de départ d'eau stérile et centrifugation 8 min à 2500 rpm. Le surnageant limpide est à 237 mOsm.

d) Lavage du culot avec 1/8 du volume de la solution de départ de NaCl 0,9%. Centrifugation 8 min à 2500 rpm. Le

surnageant limpide est à 253 mOsm. La concentration théorique calculée est alors de 64,97 mg/ml.

Cette formulation est caractérisée comme suit:

| | |
|---|---|
| pH | 5,86 |
| Osmolalité | 256 mOsm |
| [PO] | 63,51 mg/g |
| Diamètre des particules D50 (selon test T1) | 9 µm |

**Exemple 7: Obtention d'une suspension de microparticules fabriquées avec du $MgCl_2$ dans des conditions 1.**

La suspension est préparée à partir d'une solution préparée selon l'exemple 2, ayant pour caractéristiques finales 0,48 mg/g de hGH / 23,05 mg/g de PO, ajustée en pH et osmolarité.

a) Dilution à 10,0 mg/g avec du NaCl 0,9 %.
b) Floculation de la solution par ajout d'une solution de $MgCl_2$, 6 $H_2O$, 1 M, sous agitation (position n°10), avec un pousse-seringue délivrant environ 20 ml/h jusqu'à obtention d'un rapport r = 6,93. On obtient une solution à 8,59 mg/g de PO.
c) Centrifugation 10 min à 2500 rpm. Le surnageant limpide est à 654 mOsm.
d) Lavage du culot avec 1/7 du volume de la solution de départ d'eau stérile et centrifugation 10 min à 2500 rpm. Le surnageant limpide est à 291 mOsm.

Cette formulation est caractérisée comme suit :

| | |
|---|---|
| pH | 6,56 |
| Osmolalité | 323 mOsm |
| [PO] | 75,7 mg/g |
| [hGH] | 1,3 mg/g |
| Diamètre des particules D50 (selon test T1) | 8,6 µm |

**Exemple 8 : Obtention d'une suspension de microparticules fabriquées avec du $MgCl_2$ dans des conditions 2.**

La suspension est préparée à partir d'une solution préparée selon l'exemple 2, ayant pour caractéristiques finales 1,33 mg/g de hGH / 10,10 mg/g de PO, ajustée en pH et osmolarité.

a) Floculation de la solution en présence d'un balayage d'azote par ajout d'une solution de $MgCl_2$, 6 $H_2O$, 2 M bullé à l'azote, sous agitation (500 rpm), avec une pompe délivrant environ 8 ml/min jusqu'à obtention d'un rapport r = 10,01. On obtient une solution à 8,98 mg/g de PO à 914 mOsm.
b) Maturation de 3 h sous agitation.
c) Utilisation d'un module Microza (UJP-0047R - Pall) de surface spécifique égale à 0,02 $m^2$. Ce module a été au préalable conditionné (élimination du glycérol et de l'éthanol par trempage dans de l'eau), dépyrogénéisé (NaOH 7% puis rinçage à l'eau) et autoclavé.
d) Conditionnement du module avec une solution de $MgCl_2$ 900 mOsm.
e) Lavage avec 1,04 fois le volume de la formulation floculée d'eau stérile que l'on ajoute à raison de 8 ml/min afin de maintenir le volume global constant. La circulation est assurée avec une pompe à 25 % de sa puissance, en maintenant une pression de 0,3 bar. Cette étape dure 3 h. Le filtrat limpide est à 459 mOsm au final.
f) Utilisation d'un module Microza (0,02 $m^2$) dont la circulation est assurée avec une pompe à 25% de sa puissance. Concentration avec un débit de perméat de 4,4 ml/min. Cette étape dure 160 min. Le filtrat est à 324 mOsm au final.

Cette formulation, qui est une suspension fluide et blanche, possède les caractéristiques suivantes :

| | |
|---|---|
| Osmolalité | 329 mOsm |

(suite)

| | |
|---|---|
| [Mg] | 6,8 mg/g |
| [microparticules] | 43,6 mg/g |
| [hGH] | 4,56 mg/g |
| r (selon test M) | 2,30 |
| $T_r$ (selon test L) | 16 h |
| $d_{app}$ (selon test D) | 0,15 |

**Exemple 9: Obtention d'une suspension stable de microparticules fabriquées avec du $MgCl_2$ dans des conditions 3.**

a) Préparation d'une solution de PO préparée selon l'exemple 2, ci-après dénommée formulation initiale hGH 1,4 mg/g / PO 10 mg/g

Une solution d'hormone de croissance humaine recombinante est décongelée 2 h à 25 °C ([hGH] = 3,9 mg/ml, pH = 7,2, 330 mOsm).
Le polymère PO est dilué et ajusté (300 mOsm, pH = 7,4, 15,6 mg/g).
La solution d'hGH est versée sur le polymère, puis le mélange hGH/PO (1,4/10 mg/g) est dégazé.
L'association est réalisée une nuit à température ambiante.

| | | |
|---|---|---|
| PO | M (PO) | 2317,90 g |
| pH = 7,4 - 300 mOsm | [PO] | 15,61 mg/g |
| hGH | M (hGH) | 1302,30 g |
| | [hGH] | 3,9 mg/g |
| Formulation initiale | M | 3497,00 g |
| | [hGH] | 1,35 mg/g |
| | [PO] | 10,01 mg/g |

b) Préparation des microparticules hGH 5 mg/g / Mg microparticules 40 mg/g

La formulation initiale est floculée par ajout contrôlé de $MgCl_2$ 2 M avec un rapport r = 9,01, puis l'ensemble est laissé pendant 1 h (maturation).
La suspension est lavée avec de l'eau jusqu'à atteindre une osmolalité d'environ 300 mOsm par micro filtration tangentielle (module Microza de chez Pall avec une surface spécifique de 0,32 $m^2$). Elle est ensuite concentrée jusqu'à atteindre une concentration en PO comprise entre 38 et 41 mg/g.

| | | |
|---|---|---|
| Formulation initiale | M | 3458,4 g |
| | [hGH] | 1,399 mg/g |
| | [PO] | 10,01 mg/g |
| Floculation | M ($MgCl_2$, 2 M) | 457,6 g |
| | débit | 8,47 g/min |
| | M (suspension) | 3883,4g |
| | [PO] | 8,84 mg/g |
| | Osmolalité | 942 mOsm |

(suite)

| | | |
|---|---|---|
| lavage | M ($H_2O$) | 4241 g |
| | débit | 42,43 mg/min |
| | Osmolalité | 330 mOsm |
| Concentration | M (filtrat) | 3157 g |
| | débit | 39,46 mg/min |
| | M (microparticules concentrées) | 713,8 g |
| | [hGH] | 5,28 mg/g |
| | [PO] | 50,9 mg/g |
| Dilution | M ($MgCl_2$, 0,1 M) | 179,9 g |
| | M (microparticules concentrées) | 893,7 g |
| | [hGH] | 4,22 mg/g |
| | [PO] | 40,65 mg/g |

c) Préparation de la formulation mixte hGH 5 mg/g / Mg microparticules 40 mg/g / PO 23 mg/g

Les microparticules sont stabilisées par l'ajout du polymère de type PO sous sa forme lyophilisée.

Le lyophilisat est ajouté sur la suspension en agitation.

L'ensemble est mis sous vide (30 mbar) et sous agitation jusqu'au lendemain.

| | | |
|---|---|---|
| Suspension | M (microparticules concentrées) | 872,5 g |
| PO lyophilisé | [$H_2O$] | 10,125 % |
| | M ajouté | 23,175 g |
| formulation finale | M | 895,68 g |
| | [hGH] | 4,11 mg/g |
| | [PO dans microparticules] | 39,60 mg/g |
| | [PO ajouté] | 23,00 mg/g |
| | pH | 6,4 |
| | Osmolalité | 409 mOsm |
| | [Mg] | 4,0 g/l |
| | r (selon test M) | 0,94 |
| | $d_{app}$ (selon test D) | 0,14 |
| | $T_r$ (selon test L) | 30,56 h |

**Exemple 10 Préparation de particules micrométriques sèches chargées en hGH à partir d'une suspension de microparticules par un premier mode d'exécution du procédé selon l'invention.**

Une suspension de microparticules chargée en hGH est tout d'abord fabriquée dans les conditions décrites aux étapes a) à e) de l'exemple 8 (floculation, maturation, lavage). La suspension est lavée jusqu'à obtention d'une osmolalité de 280 mOsm environ. La suspension à environ 10 mg/g en polymère est ensuite lyophilisée sur un appareil de type Bioblock ALPHA 1-4 LSC après avoir été congelée dans de l'azote liquide pendant 76 h.

Reconstitution et caractérisation de la suspension :

Sur 40 mg de poudre est ajouté 1 ml d'eau PPI. La solution est agitée manuellement pendant quelques secondes

jusqu'à mouillage homogène de la poudre. La solution est laissée au repos pendant environ 10 min. Elle est homogénéisée quelques secondes par agitation manuelle et aspirée à l'aide d'une aiguille 21G. On obtient ainsi 1 ml d'une solution prête à injecter.

Les caractéristiques de la suspension sont décrites ci-dessous :

| $r = 2 \times \frac{[Mg^{2+}]}{[COO^-]}$ (selon test M) | pH | Osmolalité | $d_{app}$(mg/ml) (selon test D) |
|---|---|---|---|
| 1,4 | 6,6 | 338 | 0,10 |

**Exemple 11 Préparation de particules micrométriques sèches de polymère chargées en hGH à partir d'une suspension de microparticules par un second mode d'exécution du procédé selon l'invention.**

Une suspension de microparticules chargée en hGH est tout d'abord fabriquée dans les conditions décrites aux étapes a) à e) de l'exemple 8 (floculation, maturation, lavage). La suspension est lavée jusqu'à obtention d'une osmolalité de 280 mOsm environ. La suspension à environ 10 mg/g en polymère est ensuite séchée sur un appareil d'atomisation de type Büchi B290. La solution liquide est aspirée à une vitesse de 5 ml/min et nébulisée à travers une buse de pulvérisation alimentée en azote (7 bars - 500 l/h). Le débit d'aspiration (air de séchage) est de 40 $m^3$/h. La température d'entrée est maintenue à 90 °C, ce qui induit dans ces conditions une température en sortie de 45 °C. Dans ces conditions, la taille des particules obtenues (selon test T2) est de D(0,5) = 5 $\mu$m (50 % du volume est occupé par des particules ayant un diamètre < 5 $\mu$m).

Reconstitution et caractérisation de la suspension :

Sur 30 mg de poudre est ajouté 1 ml d'eau PPI. La solution est agitée manuellement pendant quelques secondes jusqu'à mouillage homogène de la poudre. La solution est laissée au repos pendant environ 10 min. Elle est homogénéisée quelques secondes par agitation manuelle et aspirée à l'aide d'une aiguille 21G. On obtient ainsi 1 ml d'une solution prête à injecter.

Les caractéristiques de la suspension sont décrites ci-dessous

| $r = 2 \times \frac{[Mg^{2+}]}{[COO^-]}$ (selon test M) | pH | Osmolalité | Taille D (0,5) $\mu$m (selon test T1) | $d_{app}$(mg/ml) (selon test D) |
|---|---|---|---|---|
| 2,3 | 6,8 | 598 | 10,0 | 0,15 |

**Exemple 12: Pharmacocinétique de l'hGH chez le chien après injection sous-cutanée de diverses formulations à base de polyaminoacides amphiphiles sous forme nanoparticulaire et microparticulaire.**

Douze chiens Beagle naïfs (poids de 7 à 10 kg) ont été traités avec les formulations suivantes :

| Formulation | Nombre de chiens | [hGH] (mg/ml) | [PO] (mg/ml) | Dose (mg/kg) | Dose Volume (ml/kg) |
|---|---|---|---|---|---|
| hGH IR | 4 | 4 | 0 | 1 | 0,23 |
| Formulation 1 | 4 | 5 | 22,6 | 1 | 0,22 |
| Formulation 2 | 4 | 5 | 117,5 | 1 | 0,20 |

L'hGH IR correspond à une solution d'hormone de croissance humaine recombinante ([hGH] = 4 mg/ml, pH = 7,2, 330 mOsm).

La formulation 1 est préparée suivant l'exemple 2 et la formulation 2 suivant l'exemple 7. L'hGH est dosée par un test ELISA (kit DSL 10-1900).

Les données pharmacocinétiques sont regroupées dans le tableau suivant :

| Formulation | $C_{max}$ ± SD (ng/mL) | T > 5ng/mL ± SD (h) | $AUC_{0\text{-}last}$ ± SD (ng.h/ml) | RBA (%) | $T_{50\%auc}$ ± SD (h) |
|---|---|---|---|---|---|
| hGH IR | 582 ± 155 | 18 ± 3 | 3209 ± 276 | 100 | 4 ± 1 |
| Formulation 1 | 135 ± 37 | 44 ± 6 | 2579 ± 516 | 80 | 25 ± 5 |
| Formulation 2 | 25 ± 5 | 129 ± 26 | 1759 ± 246 | 55 | 121 ± 33 |

où :

- $C_{max}$ est la concentration sérique maximale en hGH
- T > 5ng/ml est le temps où la concentration sérique en hGH est supérieure à 5 ng/ml
- AUC représente l'aire sous la courbe concentration sérique en hGH en fonction du temps
- RBA représente la biodisponibilité par rapport à une formulation *Immediate Release*
- $T_{50\%auc}$ représente le temps nécessaire pour relarguer 50% de l'hGH relarguée au total.

L'hGH IR présente un profil de libération rapide avec une concentration sérique maximale de 582 ± 155 ng/mL atteinte après un temps médian de 2 h. L'hGH est ensuite éliminée assez rapidement (T½ apparente d'environ 2 h) suivant une décroissance mono-exponentielle. L'hGH circulante n'est ensuite plus quantifiable au-delà de 24 h.

La formulation 1 présente un profil de libération prolongée de l'hGH avec une phase d'absorption lente, avant d'atteindre des concentrations sériques maximales comparables (44 ± 6 et 37 ± 4 ng/mL respectivement) après un temps médian de 24 h. La pente d'élimination indique l'absence d'hGH quantifiable au-delà de 48 h (la concentration sérique étant de 4,8 ± 3,1 ng/mL).

Cette formulation a montré ici une libération plus lente d'hGH dans le compartiment sanguin, illustrée par le décalage du Cmax centré sur 24 h. Cependant ce phénomène ne semble pas prolonger de façon significative la présence de l'hGH dans le sérum, puisque la concentration circulante à 48 h apparaît nulle. L'AUC de la formulation 1 apparaît légèrement diminuée par rapport à la référence IR : la biodisponibilité est de 80%.

La formulation 2 offre quant à elle une modification majeure du profil pharmacocinétique avec une très lente libération, suite à un temps de latence (*lag-time*) de 4 h, puis une concentration sérique maximale de 25 ± 5 ng/mL atteinte après un temps médian de 108 h (étendue : 72 - 168 h). L'allure générale de la pharmacocinétique de la formulation 2 est un profil très plat en pseudo-plateau, type perfusion *(infusion-like).* Le niveau d'hGH circulant retourne à une concentration non quantifiable entre 168 h et 240 h (7 et 10 jours). Cette formulation présente quant à elle une AUC beaucoup plus basse : perte de biodisponibilité relative de 45 % (RBA = 55%).

**Exemple 13 : Pharmacocinétique de l'hGH chez le chien après injection sous-cutanée d'une formulation à base de polyaminoacides amphiphiles sous forme microparticulaire**

Douze chiens Beagle naïfs (poids de 7 à 10 kg) ont été traités avec les formulations suivantes :

| Formulation | Nombre de chiens | [hGH] (mg/ml) | [PO] (mg/ml) | pH / mOsm | Dose (mg/kg) | Dose Volume (ml/kg) |
|---|---|---|---|---|---|---|
| hGH IR | 6 | 4,1 | 0 | 7,4 /321 | 5 x 0,1 quotidien | 0,024 |
| Formulation 3 | 6 | 4,3 | 64,2 | 6,4 /409 | 0,5 | 0,122 |

L'hGH IR correspond à une solution d'hormone de croissance humaine recombinante ([hGH] = 4,1 mg/ml, pH = 7,2, 330 mOsm). La formulation 3 est préparée suivant l'exemple 9.

Les données pharmacocinétiques sont regroupées dans le tableau suivant :

| Formulation | $C_{max}$ ± SD (ng/mL) | T > 1ng/mL ± SD (h) | $AUC_{0\text{-}last}$ ± SD (ng.h/ml) | RBA (%) | $T_{50\%auc}$ ± SD (h) |
|---|---|---|---|---|---|
| hGH IR | 90 ± 24 | 39 | 258 ± 26 | 100 | 2 ± 0,3 |
| Formulation 3 | 26 ± 16 | 108 ± 38 | 999 ± 294 | 77 | 66 ± 14 |

où :

- $C_{max}$ est la concentration sérique maximale en hGH ;
- T > 1ng/mL est le temps où la concentration sérique en hGH est supérieure à 1 ng/ml ;
- AUC représente l'aire sous la courbe concentration sérique en hGH en fonction du temps ;
- RBA représente la biodisponibilité par rapport à une formulation *Immediate Release;*
- $T_{50\%auc}$ représente le temps nécessaire pour relarguer 50% de l'hGH relarguée au total.

Les microparticules relarguent l'hGH sur plus de 5 jours avec une perte de biodisponibilité de 23%.

**Exemple comparatif 14 : Libération *in vitro* (test L) d'hGH à partir de microparticules selon l'invention et de nanoparticules de PO**

Une comparaison de la libération d'hGH à partir de nanoparticules de PO (exemple 2) et de microparticules de PO (exemple 8) est effectuée dans le test L. La phase continue est une solution tamponnée d'albumine à 30 mg/g.
On peut lire sur la figure unique annexée le temps nécessaire pour relarguer 50% de la protéine (hGH) dans les conditions de concentration où la formulation est injectable :

- nanoparticules à 23 mg/g : $t_r$ = 40 min soit 0,67 h
- microparticules à 73 mg/g : $T_r$ = 973 min soit 16,22 h

L'hGH contenue dans des microparticules se relargue 24 fois plus lentement que celle contenue dans des nanoparticules.

**Exemple 15: Préparation de flacons individuels contenant une poudre sèche lyophilisée de microparticules de polymère PO et d'insuline (100 UI/flacon)**

*Préparation de 350 g d'une solution d'insuline concentrée à 500 UI/g (17,5 mg/g) :*

6,4 g d'insuline humaine recombinante (poudre) d'activité 28,6 UI/g et contenant 4,5 % d'humidité sont introduits dans un flacon en verre. 157 g d'eau sont ajoutés et l'insuline est dispersée sous agitation magnétique lente. 46,6 g d'HCl 0,1 N sont ajoutés jusqu'à obtention d'une solution limpide d'insuline acide. 69,8 g de soude 0,1 N sont alors ajoutés de façon à obtenir une solution finale limpide ayant un pH compris entre 7 et 8. La solution est diluée à la concentration voulue par ajout de 70,2 g d'eau.

*Mésange avec la solution de polymère PO :*

326 g de la solution d'insuline concentrée précédente sont lentement versés (sous agitation magnétique) sur 3426 g de solution de polymère PO à 11 mg/g. Le mélange est filtré sur 0,2 μm et est laissé sous lente agitation pendant une nuit. Toutes les opérations qui suivent sont réalisées en conditions aseptiques.

*Floculation - lavage - concentration :*

La formulation précédente est floculée par ajout contrôlé de 377 g de $MgCl_2$ 2 M (soit, à ce stade, un rapport r = 7,2). Après 1 h de maturation,La suspension est lavée avec de l'eau jusqu'à atteindre une osmolalité d'environ 340 mOsm par microfiltration tangentielle (module Microza de chez Pall avec une surface spécifique de 0,32 $m^2$) et concentrée à 27 mg/g environ en polymère PO. Le pH est ajusté à 6,5 par ajout de soude 1 N (environ 6 g) sur la suspension. À l'issue de cette étape, la suspension a un titre en insuline d'environ 100 UI/g (la valeur exacte peut être obtenue par dosage HPLC sur colonne silice greffée C18).

*Ajout de polyvinylpyrrolidone :*

200 g de solution mère de polyvinylpyrrolidone sont préparés à 40 mg/g environ à partir de poudre de polyvinylpyrrolidone de qualité injectable (K17 par exemple) et sont filtrés sur une membrane stérilisante de 0,2 μm. 120 g de la solution ainsi filtrée sont alors ajoutés stérilement à 1200 g de la suspension précédente et le mélange est agité pendant environ 15 min.
La suspension résultant contient environ 91 UI/g d'insuline.

*Lyophilisation :*

La suspension précédente est répartie dans des flacons individuels à raison de 100 UI par flacon (soit environ 1,1 g de la suspension précédente par flacon) : les flacons sont lyophilisés stérilement pendant un cycle de 72 h. À la suite de cette étape, ils sont bouchés hermétiquement en attendant leur utilisation.

**Exemple 16 : Reconstitution des flacons de microparticules / insuline obtenus à l'exemple 15**

La suspension est reconstituée de manière extemporanée (avant utilisation) selon le protocole suivant : 1 ml d'eau est introduit à l'aide d'une seringue et d'une aiguille dans un flacon contenant 100 UI d'insuline obtenu selon l'exemple précédent.
Le flacon est agité manuellement pendant quelques secondes de façon à obtenir une suspension homogène (d'aspect laiteux) : la suspension est aspirée dans la seringue et est prête à être injectée à travers une aiguille de 30G, par exemple.
La suspension ainsi reconstituée contient :

- 23 mg/ml de polymère PO
- 100 UI/ml d'insuline (3,5 mg/ml)
- 4 mg/ml de polyvinylpyrrolidone
- 0,18 mmol/ml de $Mg^{2+}$ (soit un rapport r = 2,8)

La suspension ainsi reconstituée a un pH de 6,5 et une osmolalité de 300 mOsm.
La mesure granulométrique de la taille des particules (mesurée par le test T1) donne un diamètre médian D50 de 15 μm.
La densité apparente mesurée dans le test D est de 0,13 .
La viscosité dynamique mesurée à 20 °C est de 5 mPa.s .

**Exemple 17 : Pharmacodynamie de l'insuline chez le chien après injection sous cutanée d'une formulation à base de polyaminoacides amphiphiles sous forme microparticulaire**

Deux groupes de 6 chiens Beagle sains (poids de 10,4 ± 0,6 kg) ont été traités successivement par l'une des formulations suivantes au cours d'un essai croisé à 2 périodes :

| Formulation | Nombre de chiens | [insuline] (UI/g) | [PO] (mg/g) | Dose (UI/kg) | Dose Volume (μL/kg) |
|---|---|---|---|---|---|
| Lantus® (lot 40N300) | 12 | 100 | 0 | 1 | 10 |
| Formulation 4 | 12 | 100 | 20 | 1 | 10 |

La référence de cette étude, le Lantus®, est un analogue d'insuline modifié (insuline glargine) commercialisé par Sanofi-Aventis. La modification de deux résidus aminoacides sur la structure primaire de l'insuline humaine confère au Lantus des propriétés de libération prolongée sur une période de 24 h via une précipitation *in situ.*
La formulation 4 est préparée suivant l'exemple 16.
La glycémie est dosée par méthode enzymatique (hexokinase) sur un automate d'analyses biochimiques du sang (Advia 1650, Bayer Diagnostics).
L'analyse des résultats de pharmacodynamie est réalisée sur le pourcentage de la glycémie basale en fonction du temps.
Les résultats pharmacodynamiques sont regroupés dans le tableau suivant :

| Formulation | $C_{min}$ ± SD (%) | $APGC_{0-36h}$ ± SD (%.h) | $APGC_{formulation\ 4}$/ $APGC_{Lantus}$ ± SD (%) | $T50\%_{APGC}$ ± SD (h) |
|---|---|---|---|---|
| Lantus® | 40 ± 6 | 1250 ± 342 | - | 13,3 ± 3.1 |
| Formulation 4 | 45 ± 4 | 1389 ± 309 | 118 ± 37 | 19,7 ± 3.7 |

où :

- $C_{min}$ est le pourcentage minimum observé de la glycémie basale
- $APGC_{0-36h}$ représente la surface entre la glycémie basale et le pourcentage de la glycémie basale au cours du

temps entre 0 et 36 h post-dose

- $T_{50\%APGC}$ représente le temps nécessaire pour obtenir 50% de l'$APGC_{0-36h}$.

L'administration de la référence Lantus® conduit à une baisse rapide de la glycémie dès la première heure. L'action hypoglycémiante de l'insuline glargine est ensuite maintenue sur une période comprise entre 18 et 36 h (retour de la glycémie à son niveau basal après 30 h en moyenne).

En comparaison, l'administration de la formulation 4 a conduit également à une baisse rapide de la glycémie dès la première heure. Le pourcentage de la glycémie basale s'est ensuite maintenu en plateau jusqu'à 36 h en moyenne. La $C_{min}$ obtenue avec la formulation 4 était significativement plus haute que celle de la référence Lantus® ($p<0.005$, test t de Student unilatéral et apparié), ce qui devrait permettre de réduire considérablement les épisodes d'hypoglycémie sévère chez les patients diabétiques.

La durée d'action de la formulation 4 était nettement supérieure à celle de la référence à longue action Lantus®. Cela est illustré par une valeur de $T50\%_{APGC}$ significativement plus élevée pour la formulation 4 ($p<0.0005$, test t de Student unilatéral et apparié). Aucune perte d'$APGC_{0-36h}$ n'a été observée pour la formulation 4 par rapport à la référence Lantus®.

**Exemple 18 : Obtention de poudre lyophilisée de microparticules de polymère PO et d'interféron α -2b**

Préparation d'une solution contenant 15 mg/g de polymère et 0, 19 mg/g d'IFN

166 g de solution de polymère PO à 16,5 mg/g sont introduits dans un flacon de 500 ml. 2,3 g d'une solution à 0,3 M de méthionine sont ajoutés à la solution. Une solution congelée d'IFN-α-2b concentrée à 2,4 mg/g est décongelée à 25 °C pendant 1 h et 13 g de cette solution congelée sont introduits dans le flacon contenant la solution de polymère. Le mélange est laissé pendant 14 h à température ambiante. La solution est filtrée sur un filtre stérilisant 0,2 μm. Toutes les opérations suivantes sont réalisées en conditions aseptiques.

Floculation - lavage - concentration

129,5 g de la formulation précédente sont floculés par ajout contrôlé de 148,5 g de $MgCl_2$ 2 M (soit, à ce stade, un rapport r = 7,0 ). La suspension est répartie en 4 flacons (37 g par flacon environ) et centrifugée pendant 15 min à 3000 tr/min. 30,5 g de surnageant sont ôtés des flacons en prenant soin de ne pas toucher au culot de centrifugation. Les culots sont ensuite repris par ajout de 17 g d'eau dans chaque flacon. avec de l'eau stérile. L'osmolalité à cette étape est d'environ 300 mOsm.

Lyophilisation

La suspension est répartie en récipients de type Lyoguard® (Gore™) permettant de garder la suspension stérile lors de la lyophilisation : les récipients sont ensuite lyophilisés stérilement pendant un cycle de 72 h sur un lyophilisateur de paillasse (Christ).

**Exemple 19 : Reconstitution d'une suspension de microparticules contenant de l'interféron α -2b à partir de la poudre lyophilisée obtenue à l'exemple 18**

Afin de connaître la quantité de poudre à reprendre pour obtenir de façon précise 0,5 mg/g d'interféron dans la formulation, un essai préliminaire de reconstitution est réalisé et l'interféron α-2b est dosé en HPLC sur colonnes silice greffées C18.

La suspension est reconstituée de manière extemporanée (avant utilisation) selon le protocole suivant : 13,58 g d'eau sont ajoutés sur 1,22 g de poudre lyophilisée et la suspension est homogénéisée avec un barreau magnétique pendant 1 h.

La suspension résultante est homogène (d'aspect laiteux) : la suspension est aspirée dans la seringue et est prête à être injectée à travers une aiguille de 30G par exemple.

La suspension ainsi reconstituée contient :

- 46 mg/ml de polymère PO
- 0,5 mg/ml d'interféron α -2b
- 0,34 mmol/ml de $Mg^{2+}$ (soit un rapport r = 2,6)

La suspension ainsi reconstituée a un pH de 6,1 et une osmolalité de 705 mOsm.

**Exemple 20 : Pharmacocinétique de l'IFN chez le chien après injection sous-cutanée d'une formulation à base de polyaminoacides amphiphiles sous forme microparticulaire**

Huit chiens Beagle naïfs (poids de 9 ± 0,6 kg) ont été traités avec les formulations suivantes :

| *Formulation* | Nombre de chiens | [IFN] (mg/ml) | [PO] (mg/ml) | pH/ mOsm | Dose (μg/kg) | Dose Volume (ml/kg) |
|---|---|---|---|---|---|---|
| IFN IR | 4 | 0,5 | 0 | 6,45/ 354 | 60 | 0,12 |
| Formulation 5 | 4 | 0,5 | 46 | 6,1/ 705 | 60 | 0,12 |

L'IFN IR correspond à une solution d'interféron humain recombinant (PCGen, lot IB05.0516) ajustée en concentration, pH et osmolarité ([IFN] = 0,5 mg/ml, pH = 6,45, et 354 mOsm).
La formulation 5 est préparée suivant l'exemple 19, à partir du même lot d'interféron (PCGen).
Les résultats pharmacocinétiques sont regroupés dans le tableau suivant :

| *Formulation* | $C_{max}$ ± SD (ng/mL) | T > 50 pg/mL ± SD (h) | $AUC_{0-all}$ ± SD (ng.h/ml) | RBA (%) | $T_{50\%auc}$ ± SD (h) |
|---|---|---|---|---|---|
| IFN IR | 25,2 ± 0,4 | 22,6 ± 0,6 | 162,5 ± 27,2 | 100 | 5,1 ± 0,7 |
| Formulation 5 | 0,9 ± 0,6 | 219,8 ± 29,8 | 95,5 ± 47,5 | > 59 | 96,7 ± 31,7 |

où :

- $C_{max}$ est la concentration sérique maximale en IFN ;
- T > 50 pg/ml est le temps où la concentration sérique en IFN est supérieure à 50 pg/ml ;
- AUC représente l'aire sous la courbe concentration sérique en IFN en fonction du temps ;
- RBA représente la biodisponibilité par rapport à une formulation *Immediate Release;*
- $T_{50\%auc}$ représente le temps nécessaire pour relarguer 50% de l'IFN relargué au total.

L'IFN IR présente un profil de libération rapide avec une concentration sérique maximale de 25,2 ± 0,4 ng/mL atteinte après un temps médian de 5 h (étendue : 3 - 5 h). L'IFN circulant n'est plus quantifiable au-delà de 24 h.
La formulation 1 offre une modification majeure du profil pharmacocinétique de l'IFN avec une libération très lente, et une concentration sérique maximale de 0,9 ± 0,6 ng/mL (28 fois inférieure à celle de l'IR), atteinte après un temps médian de 108 h (étendue : 66 - 144 h). L'allure générale de la pharmacocinétique est un profil plat en pseudo-plateau. Le niveau d'IFN circulant retourne à une concentration non quantifiable entre 168 h et plus de 240 h (7 et plus de 10 jours). Cette formulation présente une AUC plus basse : perte de biodisponibilité relative de 41 % (RBA = 59%). Le T50%auc est environ 19 fois supérieur à celui de l'IFN IR.

## Revendications

**1.** Formulation pharmaceutique liquide pour la libération prolongée de PA comprenant une suspension colloïdale, aqueuse, de viscosité dynamique à 20°C inférieure ou égale à 1000 mPa.s, à base de particules micrométriques, ayant une taille comprise entre 1 et 70 μm mesurée dans un test T, composées de polymère (PO) et d'ions multivalents de polarité opposée à celle du polymère PO, dans laquelle le polymère PO

- est un copolymère amphiphile, biodégradable, hydrosoluble, où ledit copolymère est un polyaminoacide dont la chaîne principale est formée par des résidus aspartiques ou des résidus glutamiques, au moins une partie de ces résidus étant porteurs de groupements hydrophobes (GH) et de groupements hydrophiles ionisables (GI), au moins en partie ionisés,
- et forme spontanément une suspension colloïdale de nanoparticules dans l'eau, à pH = 7,0, en condition isotonique,

lesdites nanoparticules étant aptes à s'associer spontanément de façon non covalente avec au moins un principe actif (PA), à pH = 7,0, en condition isotonique ;

- les ions multivalents de ladite formulation sont :
- de valence inférieure ou égale à 4, de préférence des ions divalents, des ions trivalents ou leurs combinaisons,
- de polarité opposée à celle des groupements ionisables GI du polymère PO,
- dans une quantité telle que le rapport r, mesuré dans un test M, est compris entre 0,3 et 10, de préférence entre 0,6 et 5,0 et, plus préférentiellement encore, entre 0,8 et 3,0, ledit rapport r répondant à la formule $r = n \times \frac{[IM]}{[GI]}$, où

  - n est la valence desdits ions multivalents,
  - [IM] est la concentration molaire en ions multivalents,
  - [GI] est la concentration molaire en groupements ionisables GI.

**2.** Formulation selon la revendication 1, **caractérisée en ce que** les particules micrométriques ont une densité apparente $d_{app}$ en polymère, mesurée dans un test D, comprise entre 0,05 et 1,0, de préférence entre 0,07 et 0,7, de préférence entre 0,1 et 0,5.

**3.** Formulation selon la revendication 1, **caractérisée par** un accroissement de la durée $T_r$ de libération d'un PA donné, tel que mesuré dans un test L, par rapport à la durée $t_r$ de libération mesurée dans le même test L, d'une formulation identique injectable ne contenant pas d'ions multivalents, cet accroissement étant tel que $T_r$ est supérieur ou égal à $1,1 \times t_r$, et, de préférence, tel que $T_r$ est supérieur ou égal à $1,5 \times t_r$.

**4.** Formulation selon la revendication 1, **caractérisée en ce qu'**elle comprend au moins un PA associé aux microparticules de PO.

**5.** Formulation selon la revendication 1, **caractérisée en ce que** le polymère modifié hydrophobe PO est défini par la formule générale (I) suivante :

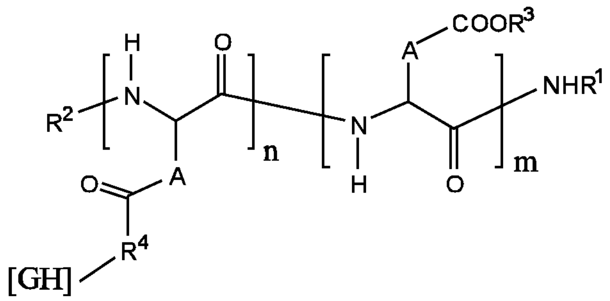

(I)

dans laquelle :

- $R^1$ représente un H, un alkyle linéaire en C2 à C10 ou ramifié en C3 à C10, un radical benzyle, un résidu acide aminé terminal, ou $-R^4$-[GH] ;
- $R^2$ représente un H, un groupe acyle linéaire en C2 à C10 ou ramifié en C3 à C10, un pyroglutamate ou $-R^4$-[GH] ;
- $R^3$ est un H ou un cation métallique choisi parmi le sodium ou le potassium ;
- $R^4$ représente une liaison directe ou un espaceur à base de 1 à 4 résidus acide aminé ;
- A représente indépendamment un radical $-CH_2-$ (résidu aspartique) ou $-CH_2-CH_2-$ (résidu glutamique) ;
- n/(n+m) est défini comme le taux de greffage molaire et sa valeur est suffisamment basse pour que PO mis en solution dans l'eau à pH = 7 et à 25 °C forme une suspension colloïdale de particules submicroniques de PO, de préférence n/(n + m) est compris entre 1 et 25 % molaire et mieux encore entre 1 et 15 % molaire;
- n + m varie de 10 à 1000, de préférence entre 50 et 300 ;
- GH représente un groupement hydrophobe.

**6.** Formulation selon la revendication 1, **caractérisée en ce que** le (ou les) PO répond(ent) à l'une des formules générales (II), (III) et (IV) suivantes :

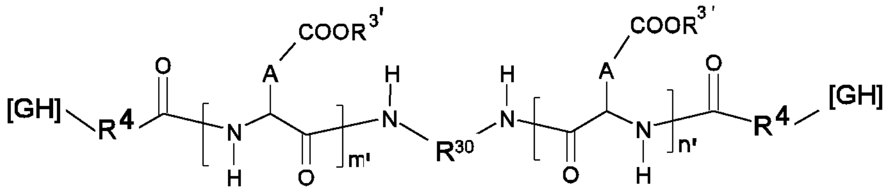

(II)

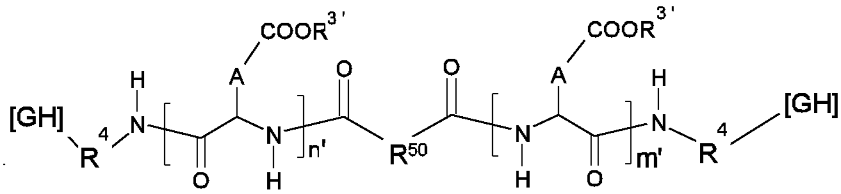

(III)

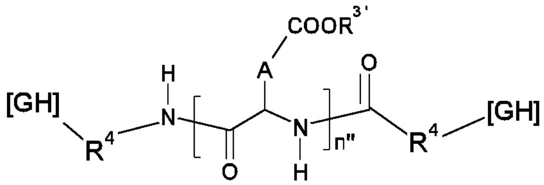

(IV)

dans lesquelles :

- GH représente un groupement hydrophobe ;
- $R^{30}$ est un groupement alkyle linéaire en C2 à C6 ;
- $R^{3'}$ est un H ou un cation métallique choisi parmi le sodium ou le potassium ;
- $R^{50}$ est un groupement alkyle, dialcoxy ou diamine en C2 à C6 ;
- $R^4$ représente une liaison directe ou un espaceur à base de 1 à 4 résidus acide aminé ;
- A représente indépendamment un radical $-CH_2-$ (résidu aspartique) ou $-CH_2-CH_2-$ (résidu glutamique) ;
- (n' + m') ou n" est défini comme le degré de polymérisation et varie de 10 à 1000, de préférence entre 50 et 300.

**7.** Formulation selon la revendication 1, **caractérisée en ce que** PO présente des groupements GI anioniques et **en ce que** les ions multivalents sont des cations multivalents, de préférence des cations divalents, plus préférentiellement encore choisis dans le groupe comprenant : $Mg^{2+}$, $Ca^{2+}$, $Zn^{2+}$, $Fe^{2+}$, $Cu^{2+}$ ou leurs mélanges, ou des cations trivalents, plus préférentiellement encore choisis dans le groupe comprenant : $Al^{3+}$, $Fe^{3+}$ ou leurs mélanges.

**8.** Formulation selon l'une quelconque des revendications 4 à 7, **caractérisée en ce que** le PA est l'hormone de croissance humaine recombinante hGH.

**9.** Formulation selon l'une quelconque des revendications 4 à 7, **caractérisée en ce que** le PA est l'insuline.

**10.** Formulation selon l'une quelconque des revendications 4 à 7, **caractérisée en ce que** le PA est l'interferon $\alpha$-2b.

**11.** Procédé de préparation de la formulation selon l'une quelconque des revendications précédentes, **caractérisé en ce qu'**il consiste essentiellement :

a. à mettre en oeuvre ou préparer une suspension colloïdale de nanoparticules d'au moins un PO;
b. éventuellement, à mélanger cette suspension colloïdale de nanoparticules de PO avec au moins un PA, de préférence en solution aqueuse;
c. éventuellement à filtrer la suspension ainsi obtenue ;
d. à ajouter des ions multivalents (de préférence sous forme de sel(s)) de polarité opposée à celle des groupements GI du polymère PO, lesdits ions multivalents étant ajoutés en quantité telle que le rapport r,

répondant à la formule $r = n \times \frac{[IM]}{[GI]}$, où

- n est la valence desdits ions multivalents,
- [IM] est la concentration molaire en ions multivalents,
- [GI] est la concentration molaire en groupements ionisables GI,

est compris entre 0,3 et 10, de préférence entre 0,6 et 5,0 et, plus préférentiellement encore, entre 0,8 et 3,0;
e. au besoin à ajuster le pH ou l'osmolarité.

**12.** Procédé de préparation d'un produit dérivé de la formulation selon l'une quelconque des revendications 1 à 10 ou de la formulation obtenue par le procédé selon la revendication 11, **caractérisé en ce qu'**il consiste essentiellement à sécher la suspension de particules micrométriques, de manière à obtenir une forme solide, de préférence une poudre de particules micrométriques, susceptible d'être stockée ou administrée.

**13.** Procédé de préparation de la formulation selon l'une quelconque des revendications 1 à 10, **caractérisé en ce qu'**il consiste essentiellement :

- à mettre en oeuvre au moins un produit dérivé chargé ou non en PA obtenu par le procédé selon la revendication 11,
- et à mélanger ce produit dérivé avec de l'eau ou une solution aqueuse S de reconstitution.

**14.** Produit dérivé de la formulation selon l'une quelconque des revendications 1 à 10 **caractérisé en ce qu'**il est sous forme non liquide et **en ce qu'**il comprend des particules micrométriques de polymère (PO), ayant une taille comprise entre 1 et 70 μm mesurée dans un test T,

i. le polymère PO

- étant un copolymère amphiphile, biodégradable, hydrosoluble , où ledit copolymère est un polyaminoacide dont la chaîne principale est formée par des résidus aspartiques ou des résidus glutamiques, au moins une partie de ces résidus étant porteurs de groupements hydrophobes (GH) et de groupements hydrophiles ionisables (GI),
- et formant spontanément une suspension colloïdale de nanoparticules dans l'eau, à pH = 7,0, en condition isotonique,

ii. lesdites particules étant aptes à s'associer spontanément de façon non covalente avec au moins un principe actif (PA), à pH = 7,0, en condition isotonique;

et **en ce que** ledit produit contient des dérivés d'ions multivalents, de préférence d'ions divalents, de polarité opposée à celle des groupements GI du polymère, le rapport r étant compris entre 0,3 et 10, de préférence entre 0,6 et 5,0 et, plus préférentiellement encore entre 0,8 et 3,0,

et répondant à la formule $r = n \times \frac{[IM]}{[GI]}$, où

- n est la valence desdits ions multivalents,
- [IM] est la concentration molaire en ions multivalents,
- [GI] est la concentration molaire en groupements ionisables GI.

**15.** Procédé de préparation de médicaments, en particulier pour administration parentérale, muqueuse, sous-cutanée, intramusculaire, intradermique, intrapéritonéale, intracérébrale ou dans une tumeur, voire par voie orale, nasale, pulmonaire, vaginale ou oculaire, **caractérisé en ce qu'**il consiste essentiellement à mettre en oeuvre au moins une formulation selon l'une quelconque des revendications 1 à 10 et/ou une formulation obtenue par le procédé selon la revendication 11 ou 12 et/ou tout produit dérivé et/ou tout précurseur de ladite formulation.

**Patentansprüche**

1. Flüssige pharmazeutische Formulierung zur verzögerten Freisetzung von PA, umfassend eine wässrige kolloidale Suspension mit einer dynamischen Viskosität bei 20°C von weniger als oder gleich 1000 mPa.s auf der Basis mikrometrischen Partikeln mit einer, in einem Test T gemessenen, Größe zwischen 1 und 70 μm, die aus dem Polymer (PO) bestehen, und mehrwertige Ionen mit einer Polarität, die derjenigen des Polymers PO entgegengesetzt ist, wobei das Polymer PO

   - ein amphiphiles, biologisch abbaubares, wasserlösliches Copolymer ist, wobei das Copolymer eine Polyaminosäure ist, deren Hauptkette von Asparaginsäureresten oder Glutaminsäureresten gebildet wird, wobei mindestens ein Teil dieser Reste hydrophobe Gruppen (GH) und ionisierbare hydrophile Gruppen (GI) trägt, die mindestens zum Teil ionisiert sind,
   - und spontan eine kolloidale Suspension von Nanopartikeln in Wasser bei pH = 7,0 unter isotonischen Bedingungen bildet,

   wobei die Nanopartikeln dazu fähig sind, spontan und nichtkovalent mit mindestens einem Wirkstoff (PA) bei pH = 7,0 unter isotonischen Bedingungen zu assoziieren,

   - die mehrwertigen Ionen der Formulierung:
   - eine Wertigkeit von weniger als oder gleich 4 haben, vorzugsweise zweiwertige Ionen, dreiwertige Ionen oder deren Kombinationen sind,
   - eine zu derjenigen der ionisierbaren Gruppen GI des Polymers entgegengesetzte Polarität haben,
   - in einer derartigen Menge vorliegen, dass das, in einem Test M gemessene, Verhältnis r zwischen 0,3 und 10, vorzugsweise zwischen 0,6 und 5,0, noch bevorzugter zwischen 0,8 und 3,0 beträgt, wobei das Verhältnis r der folgenden Formel entspricht $r = n \times \frac{[IM]}{[GI]}$, worin

   - n die Wertigkeit der mehrwertigen Ionen ist,
   - [IM] die molare Konzentration an mehrwertigen Ionen ist,
   - [GI] die molare Konzentration an ionisierbaren Gruppen GI ist.

2. Formulierung nach Anspruch 1, **dadurch gekennzeichnet, dass** die mikrometrischen Partikeln eine, in einem Test D gemessene, Polymer scheinbare Dichte $d_{app}$ zwischen 0,05 und 1,0, vorzugsweise zwischen 0,07 und 0,7, vorzugsweise zwischen 0,1 und 0,5 haben.

3. Formulierung nach Anspruch 1, **gekennzeichnet durch** eine Zunahme der Dauer $T_r$ der Freisetzung eines gegebenen PA, wie in einem Test L gemessen, im Verhältnis zu der Dauer $t_r$ der Freisetzung, wie in dem gleichen Test L gemessenen, einer identischen injizierbaren Formulierung, die keine mehrwertigen Ionen enthält, wobei diese Zunahme derart ist, dass $T_r$ größer als oder gleich $1,1 \times t_r$ ist, und vorzugsweise derart, dass $T_r$ größer als oder gleich $1,5 \times t_r$ ist.

4. Formulierung nach Anspruch 1, **dadurch gekennzeichnet, dass** sie mindestens einen mit den PO-Mikropartikeln assoziierten PA umfasst.

5. Formulierung nach Anspruch 1, **dadurch gekennzeichnet, dass** hydrophobe modifizierte Polymer PO durch die folgende allgemeine Formel (I) definiert ist:

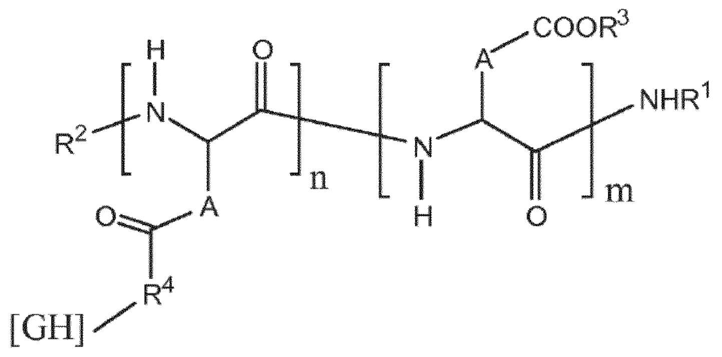

(I)

worin:

- $R^1$ für einen H, ein lineares C2- bis C10-Alkyl oder verzweigtes C3- bis C10-Alkyl, einen Benzylrest, einen terminalen Aminosäurerest oder -$R^4$-[GH] steht;
- $R^2$ für einen H, eine lineare C2- bis C10-Acylgruppe oder verzweigte C3- bis C10-Acylgruppe, ein Pyroglutamat oder -$R^4$-[GH] steht;
- $R^3$ für einen H oder ein Metallkation steht, das aus Natrium oder Kalium ausgewählt ist;
- $R^4$ für eine direkte Bindung oder einen Spacer auf der Basis von 1 bis 4 Aminosäureresten steht;
- A unabhängig für einen -$CH_2$- (Asparaginsäurerest) oder -$CH_2$-$CH_2$-Rest (Glutaminsäurerest) steht;
- n/(n + m) als der molare Pfropfungsgrad definiert ist und sein Wert ausreichend niedrig ist, dass PO in Lösung in Wasser bei pH = 7 und bei 25°C eine kolloidale Suspension von PO-Submikronpartikeln bildet, vorzugsweise beträgt n/(n + m) zwischen 1 und 25 mol% und noch besser zwischen 1 und 15 mol%;
- n + m von 10 bis 1000, vorzugsweise zwischen 50 und 300, variiert;
- GH für eine hydrophobe Gruppe steht.

6. Formulierung nach Anspruch 1, **dadurch gekennzeichnet, dass** das (oder die) PO einer der folgenden allgemeinen Formeln (II), (III) oder (IV) entspricht/entsprechen:

(II)

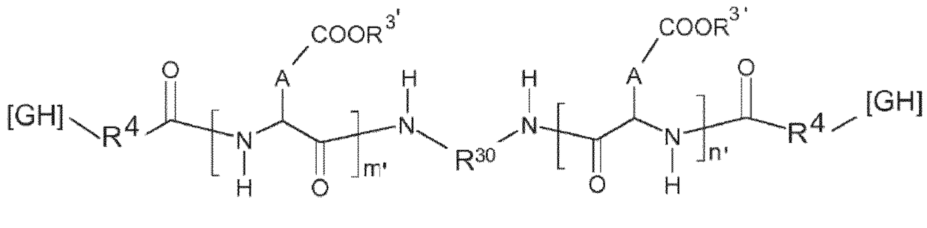

(III)

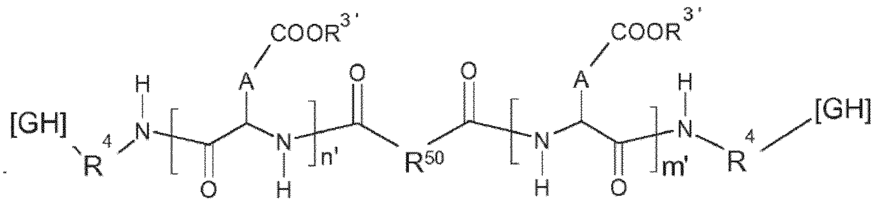

(IV)

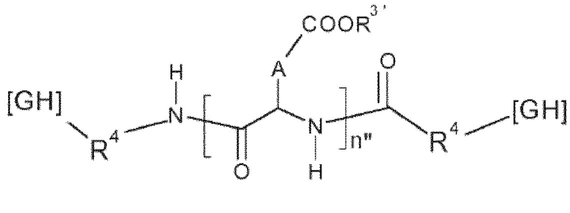

in denen:

- GH für eine hydrophobe Gruppe steht;
- $R^{30}$ eine lineare C2- bis C6-Alkylgruppe ist;
- $R^{3'}$ ein H oder ein aus Natrium oder Kalium ausgewähltes Metallkation ist;
- $R^{50}$ eine C2- bis C6-Alkyl-, -Dialkoxy- oder -Diamingruppe ist;
- $R^4$ für eine direkte Bindung oder einen Spacer auf der Basis von 1 bis 4 Aminosäureresten steht;
- A unabhängig für einen $-CH_2-$ (Asparaginsäurerest) oder $-CH_2-CH_2-$Rest (Glutaminsäurerest) steht;
- (n' + m') oder n'' als der Polymerisationsgrad definiert ist und von 10 bis 1000, vorzugsweise zwischen 50 und 300, variiert.

**7.** Formulierung nach Anspruch 1, **dadurch gekennzeichnet, dass** PO anionische Gruppen GI aufweist, und dadurch, dass die mehrwertigen Ionen mehrwertige Kationen, vorzugsweise zweiwertige Kationen, noch bevorzugter aus der Gruppe ausgewählt sind, die Folgende umfasst: $Mg^{2+}$, $Ca^{2+}$, $2n^{2+}$, $Fe^{2+}$, $Cu^{2+}$ oder deren Mischungen, oder dreiwertige Kationen, noch bevorzugter aus der Gruppe ausgewählt sind, die Folgende umfasst: $Al^{3+}$, $Fe^{3+}$ oder deren Mischungen.

**8.** Formulierung nach einem der Ansprüche 4 bis 7, **dadurch gekennzeichnet, dass** der PA rekombinantes menschliches Wachstumshormon hGH ist.

**9.** Formulierung nach einem der Ansprüche 4 bis 7, **dadurch gekennzeichnet, dass** der PA das Insulin ist.

**10.** Formulierung nach einem der Ansprüche 4 bis 7, **dadurch gekennzeichnet, dass** der PA Interferon $\alpha$-2b ist.

**11.** Verfahren zur Herstellung der Formulierung nach einem der vorhergehenden Ansprüche, **dadurch gekennzeichnet, dass** es im Wesentlichen aus Folgenden besteht:

a. Verwenden oder Herstellen einer kolloidalen Suspension von Nanopartikeln mindestens eines PO,
b. gegebenenfalls Mischen dieser kolloidalen Suspension von PO-Nanopartikeln mit mindestens einem PA, vorzugsweise in wässriger Lösung,
c. gegebenenfalls Filtrieren der so erhaltenen Suspension,

d. Zugeben von mehrwertigen Ionen (vorzugsweise in Form eines Salzes bzw. von Salzen mit einer Polarität, die zu derjenigen der Gruppen GI des Polymers PO entgegengesetzt ist, wobei die mehrwertigen Ionen in einer derartigen Menge zugegeben werden, dass das Verhältnis r, das der folgenden Formel entspricht $r = n \times \frac{[IM]}{[GI]}$, worin

- n die Wertigkeit der mehrwertigen Ionen ist,
- [IM] die molare Konzentration an mehrwertigen Ionen ist,
- [GI] die molare Konzentration an ionisierbaren Gruppen GI ist,

zwischen 0,3 und 10, vorzugsweise zwischen 0,6 und 5,0, noch bevorzugter zwischen 0,8 und 3,0 beträgt;
e. nach Bedarf Anpassen des pH-Werts oder der Osmolarität.

**12.** Verfahren zur Herstellung eines von der Formulierung nach einem der Ansprüche 1 bis 10 oder von der durch das Verfahren nach Anspruch 11 erhaltenen Formulierung abgeleiteten Produkts, **dadurch gekennzeichnet, dass** es im Wesentlichen aus dem Trocknen der Suspension von mikrometrischen Partikeln besteht, um eine feste Form, vorzugsweise ein Pulver, von mikrometrischen Partikeln zu erhalten, die aufbewahrt oder verabreicht werden kann.

**13.** Verfahren zur Herstellung der Formulierung nach einem der Ansprüche 1 bis 10, **dadurch gekennzeichnet, dass** es im Wesentlichen aus Folgendem besteht:

- Verwenden mindestens eines durch das Verfahren nach Anspruch 11 erhaltenen, mit PA beladenen oder nicht beladenen, abgeleiteten Produkts
- und Mischen dieses abgeleiteten Produkts mit Wasser oder einer wässrigen Rekonstitutionslösung S.

**14.** Von der Formulierung nach einem der Ansprüche 1 bis 10 abgeleitetes Produkt, **dadurch gekennzeichnet, dass**

es in nicht-flüssiger Form vorliegt und dass es mikrometrischen Partikeln des Polymers (PO) mit einer in einem Test T gemessenen Größe zwischen 1 und 70 μm umfasst,

i. wobei das Polymer PO

- ein amphiphiles, biologisch abbaubares, wasserlösliches Copolymer ist, wobei das Copolymer eine Polyaminosäure ist, deren Hauptkette von Asparaginsäureresten oder Glutaminsäureresten gebildet wird, wobei mindestens ein Teil dieser Reste hydrophobe Gruppen (GH) und ionisierbare hydrophile Gruppen (GI) trägt,
- und spontan eine kolloidale Suspension von Nanopartikeln in Wasser bei pH = 7,0 unter isotonischen Bedingungen bildet,

ii. wobei die Nanopartikeln dazu fähig sind, spontan und nichtkovalent mit mindestens einem Wirkstoff (PA) bei pH - 7,0 unter isotonischen Bedingungen zu assoziieren,

und dadurch, dass das Produkt Derivate von mehrwertigen Ionen, vorzugsweise von zweiwertigen Ionen, mit einer zu derjenigen der Gruppen GI des Polymers entgegengesetzten Polarität enthalten, wobei das Verhältnis r zwischen 0,3 und 10, vorzugsweise zwischen 0,6 und 5,0 und noch bevorzugter zwischen 0,8 und 3,0 beträgt und der folgenden Formel entspricht $r = n \times \frac{[IM]}{[GI]}$,

worin

- n die Wertigkeit der mehrwertigen Ionen ist,
- [IM] die molare Konzentration an mehrwertigen Ionen ist,
- [GI] die molare Konzentration an ionisierbaren Gruppen GI ist.

**15.** Verfahren zur Herstellung von Arzneimitteln, insbesondere für die parenterale, mukosale, subkutane, intramuskuläre, intradermale, intraperitoneale, intrazerebrale Verabreichung oder die Verabreichung in einen Tumor sogar auf oralem, nasalem, pulmonalem, vaginalem oder okularem Weg, **dadurch gekennzeichnet, dass** es im Wesentlichen aus der Verwendung mindestens einer Formulierung nach einem der Ansprüche 1 bis 10 und/oder einer durch das Verfahren nach Anspruch 11 oder 12 erhaltenen Formulierung und/oder jedes beliebigen abgeleiteten Produkts und/oder jedes beliebigen Vorläufers der Formulierung besteht.

## Claims

**1.** Liquid pharmaceutical formulation for the prolonged release of AP comprising an aqueous colloidal suspension of dynamic viscosity at 20°C less than or equal to 1000 mPa.s, based on micrometric particles, having a size of between 1 and 70 μm as measured in a test T, comprised of polymer (PO) and multivalent ions of opposite polarity to that of the polymer PO, wherein the polymer PO

- is a water-soluble, biodegradable, amphiphilic copolymer, wherein said copolymer is a polyamino acid whose main chain is made up of aspartic residues or glutamic residues, at least some of these residues carrying hydrophobic groups (GH) and ionizable hydrophilic groups (GI) at least partially ionized,
- and spontaneously forms a colloidal suspension of nanoparticles in water, at pH = 7.0, under isotonic conditions,

said nanoparticles being capable of associating spontaneously and non-covalently with at least one active principle (AP), at pH = 7.0, under isotonic conditions;

- the multivalent ions of said formulation are:

  - of valency less than or equal to 4, preferably divalent ions, trivalent ions or mixtures thereof,
  - of opposite polarity to that of the ionizable groups GI of the polymer PO,
  - in an amount such that the ratio r, measured in a test M, is comprised between 0.3 and 10, preferably between 0.6 and 5.0 and even more preferably between 0.8 and 3.0, said ratio r being defined by the

formula $r = n \times \frac{[IM]}{[GI]}$, where

- n is the valency of said multivalent ions,
- [IM] is the molar concentration of multivalent ions,
- [GI] is the molar concentration of ionizable groups GI.

2. Formulation according to claim 1, **characterized in that** the micrometric particles have an apparent polymer density $d_{app}$, measured in a test D, comprised between 0.05 and 1.0, preferably of between 0.07 and 0.7 and particularly preferably of between 0.1 and 0.5.

3. Formulation according to claim 1, **characterized by** an increase in the release time $T_r$ of a given AP, as measured in a test L, relative to the release time $t_r$ as measured in the same test L, of an identical injectable formulation not containing multivalent ions, this increase being such that $T_r$ is greater than or equal to 1.1 x $t_r$ and, preferably, such that $T_r$ is greater than or equal to $1.5 \times t_r$.

4. Formulation according to claim 1, **characterized in that** it comprises at least one AP associated with the microparticles of PO.

5. Formulation according to claim 1, **characterized in that** the hydrophobically modified polymer PO is defined by general formula (I) below:

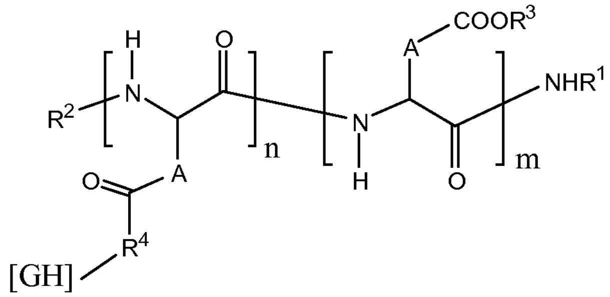

(I)

wherein:

- $R^1$ is H, a linear C2 to C10 or branched C3 to C10 alkyl, a benzyl radical, a terminal residue of amino acid, or $-R^4$-[GH];
- $R^2$ is H, a linear C2 to C10 or branched C3 to C10 acyl group, a pyroglutamate or $-R^4$-[GH];
- $R^3$ is H or a metal cation chosen from sodium or potassium,
- $R^4$ is a direct bond or a spacer based on 1 to 4 amino acid residues;
- A independently is a radical $-CH_2-$ (aspartic residue) or $-CH_2-CH_2-$ (glutamic residue);
- n/(n + m) is defined as the molar grafting rate and its value is sufficiently low for PO dissolved in water at pH = 7 and at 25°C to form a colloidal suspension of submicronic particles of PO, preferably n/(n + m) is comprised between 1 and 25 mol% and even better between 1 and 15 mol%;
- n + m varies from 10 to 1000, preferably between 50 and 300; and
- GH is a hydrophobic group.

6. Formulation according to claim 1, **characterized in that** the PO has (have) one of general formulae (II), (III) and (IV) below:

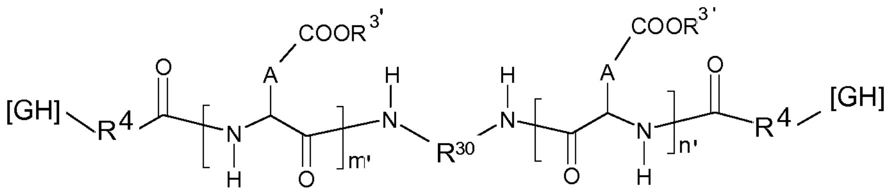

**(II)**

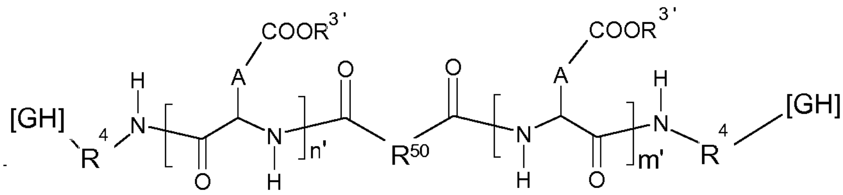

**(III)**

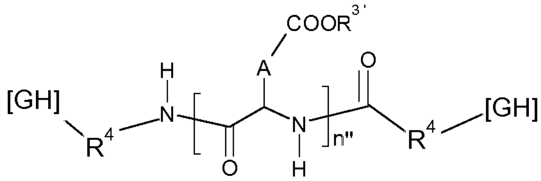

**(IV)**

wherein:

- GH is a hydrophobic group;
- $R^{30}$ is a linear C2 to C6 alkyl group;
- $R^{3'}$ is H or a metal cations chosen from sodium or potassium;
- $R^{50}$ is a C2 to C6 alkyl, dialkoxy or diamine group;
- $R^4$ is a direct bond or a spacer based on 1 to 4 amino acid residues;
- A independently is a radical $-CH_2-$ (aspartic residue) or $-CH_2-CH_2-$ (glutamic residue);
- (n' + m') or n" is defined as the degree of polymerization and varies from 10 to 1000 and preferably between 50 and 300.

**7.** Formulation according to claim 1, **characterized in that** PO has anionic groups GI and **in that** the multivalent ions are multivalent cations, preferably divalent cations and particularly preferably ones selected from the group comprising: $Mg^{2+}$, $Ca^{2+}$, $Zn^{2+}$, $Fe^{2+}$, $Cu^{2+}$ or mixtures thereof, or trivalent cations, more preferably chosen from the group comprising: $Al^{3+}$, $Fe^{3+}$ and mixtures thereof.

**8.** Formulation according to any one of claims 4 to 7, **characterized in that** the AP is the recombinant human growth hormone hGH.

**9.** Formulation according to any one of claims 4 to 7, **characterized in that** the AP is insulin.

**10.** Formulation according to any one of claims 4 to 7, **characterized in that** the AP is interferon $\alpha$-2b.

**11.** Process for the preparation of the formulation according to any one of the preceding claims, **characterized in that** it consists essentially in:

a. taking or preparing a colloidal suspension of nanoparticles of at least one PO,
b. optionally mixing this colloidal suspension of nanoparticles of PO with at least one AP, preferably in aqueous solution,
c. optionally filtering the resulting suspension,
d. adding multivalent ions (preferably in the form of salt(s)) of opposite polarity to that of the groups GI of the polymer PO, said multivalent ions being added in an amount such that the ratio r,

defined by the formula $r = n \times \frac{[IM]}{[GI]}$, where

- n is the valency of said multivalent ions,
- [IM] is the molar concentration of multivalent ions,
- [GI] is the molar concentration of ionizable groups GI,

is between 0.3 and 10, preferably between 0.6 and 5.0 and even more preferably between 0.8 and 3.0, and
e. if necessary, adjusting the pH or the osmolarity.

**12.** Process for the preparation of a product derived from the formulation according to any one of claims 1 to 10 or from the formulation obtained by the process according to claim 11, **characterized in that** it consists essentially in drying the suspension of micrometric particles, in order to obtain a solid form, preferably a powder of micrometric particles, capable of being stored or administered.

**13.** Process for the preparation of the formulation according to any one of claims 1 to 10, **characterized in that** it consists essentially in:

- taking at least one derived product loaded or not loaded with AP obtained by the process according to claim 11,
- and mixing this derived product with water or an aqueous solution S of reconstitution.

**14.** Product derived from the formulation according to any one of claims 1 to 10, **characterized in that** it is in non-liquid form and **in that** it comprises micrometric particles of polymer (PO), having a size of between 1 and 70 μm as measured in a test T,

i. the polymer PO

- being a water-soluble, biodegradable, amphiphilic copolymer, wherein said copolymer is a polyamino acid whose main chain is made up of aspartic residues or glutamic residues, at least some of these residues carrying hydrophobic groups (GH) and ionizable hydrophilic groups (GI) at least partially ionized,
- and spontaneously forming a colloidal suspension of nanoparticles in water, at pH = 7.0, under isotonic conditions,

ii. said particles being capable of associating spontaneously and non-covalently with at least one active principle (AP), at pH = 7.0, under isotonic conditions;

these micrometric particles of PO, preferably of between 2 and 40 μm;
and **in that** said product contains derivatives of multivalent ions, preferably divalent ions, of opposite polarity to that of the groups GI of the polymer, the ratio r being comprised between 0.3 and 10, preferably between 0.6 and 5.0 and even more preferably between 0.8 and 3.0,

and being defined by the formula $r = n \times \frac{[IM]}{[GI]}$, where

- n is the valency of said multivalent ions,
- [IM] is the molar concentration of multivalent ions,
- [GI] is the molar concentration of ionizable groups GI.

**15.** Process for the preparation of drugs, particularly for administration by the parenteral, mucosal, subcutaneous, intramuscular, intradermal, intraperitoneal or intracerebral route or into a tumor, or by the oral, nasal, pulmonary, vaginal or ocular route, **characterized in that** it consists essentially in using at least one formulation according to any one of claims 1 to 10 and/or a formulation obtained by the process according to any one of claims 11 or 12, and/or any product derived from, and/or any precursor of said formulation.

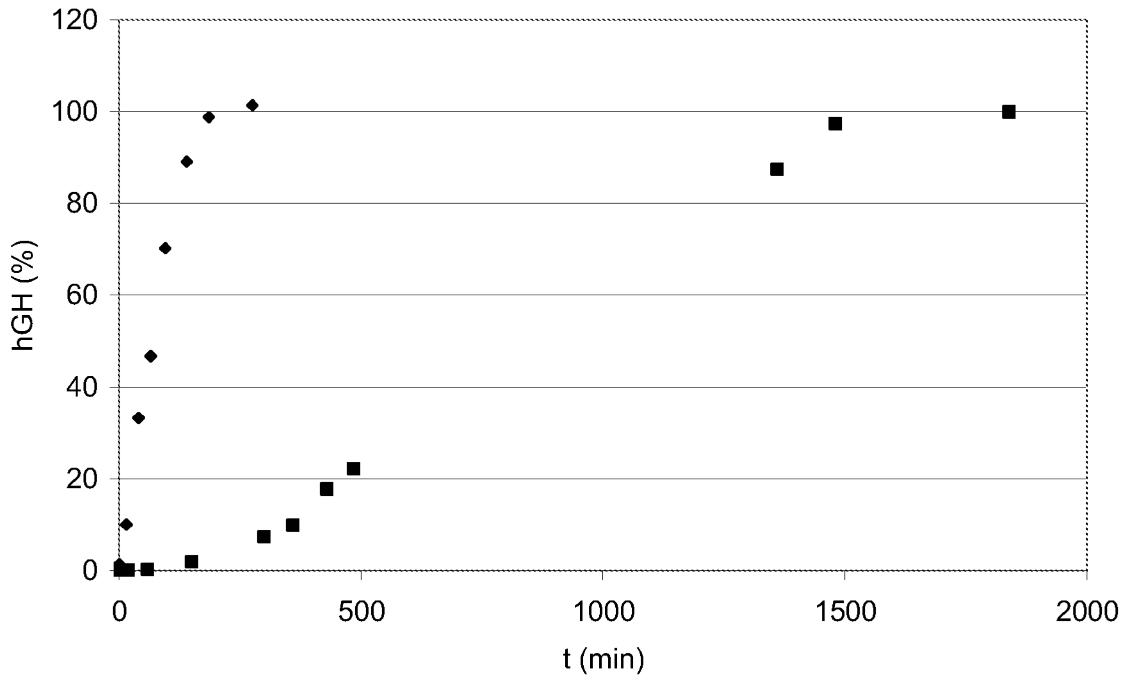


FIGURE UNIQUE

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5904936 B
- WO 2005033181 A
- WO A05051416 A
- WO 05051416 A
- WO A0030618 A
- FR 2801226 A
- WO 0030618 A


### Littérature non-brevet citée dans la description


- Surfactant Science Series. Surfactant Solutions. M. Dekker, 1984, vol. 22
- **TOMIDA et al.** *Polymer,* 1997, vol. 38, 4733-36
- **P.K. GUPTA et al.** Injectable Drug Development. Interpharm Press, 1999